# EUROPÄISCHE PATENTSCHRIFT

(11) **EP 2 424 603 B1**
(45) Veröffentlichungstag und Bekanntmachung des Hinweises auf die Patenterteilung: **31.12.2014**
(21) Anmeldenummer: 10717049.0
(22) Anmeldetag: 15.04.2010
(51) Int. Cl.: A61M 15/00

(54) **INHALATIONSVORRICHTUNG**
INHALATION DEVICE
DISPOSITIF D'INHALATION

(30) Priorität: 28.04.2009 EP 09005863
(43) Veröffentlichungstag der Anmeldung: 07.03.2012
(73) Patentinhaber: Boehringer Ingelheim International GmbH, 55216 Ingelheim am Rhein (DE)
(72) Erfinder: WACHTEL, Herbert, 55216 Ingelheim am Rhein (DE); BICKMANN, Deborah, 55216 Ingelheim am Rhein (DE); LINZ, Sarah, 55216 Ingelheim am Rhein (DE)
(74) Vertreter: Simon, Elke Anna Maria
(86) Internationale Anmeldenummer: PCT/EP2010/002315
(87) Internationale Veröffentlichungsnummer: WO 2010/124795

(56) Entgegenhaltungen:
- EP-A1- 1 884 254
- EP-A2- 0 965 355
- WO-A1-03/068299
- WO-A2-2008/124666
- DE-C1- 3 513 628
- DE-U1- 20 018 588

## Beschreibung

Die vorliegende Erfindung betrifft eine Inhalationsvorrichtung gemäß dem Oberbegriff des Anspruchs 1.

Die vorliegende Erfindung betrifft insbesondere eine Inhalationsvorrichtung zur sogenannten Soft-Mist-Inhalation, bei der ein sich nur verhältnismäßig langsam ausbreitender Sprühnebel (Aerosol) erzeugt wird. Derartige Sprühnebel im Sinne der vorliegenden Erfindung werden insbesondere mit einer Geschwindigkeit von weniger als 2 m/s, bevorzugt weniger als 1,5 m/s und ganz besonders bevorzugt weniger als 1 m/s (jeweils gemessen in einem Abstand von 10 cm von einer Austragsdüse), ausgegeben.

Bei der Inhalation einer zerstäubten Arzneimittelzubereitung bzw. eines Aerosols ist ein möglichst hoher Feinpartikelanteil wesentlich bzw. wünschenswert, da im wesentlichen nur solche Partikel die gewünschten Regionen der Lunge beim Inhalieren erreichen. Der Begriff "Feinpartikelanteil" bezeichnet bei der vorliegenden Erfindung den Anteil der Partikel, die einen aerodynamischen Durchmesser von 5 µm oder weniger aufweisen, bezogen auf die insgesamt ausgegebene Menge.

Problematisch ist bei Inhalatoren bzw. Zerstäubern generell, daß das Auslösen der Zerstäubung einer Arzneimittelzubereitung und das Einatmen koordiniert werden müssen. Dies kann für den einzelnen Benutzer schwierig sein. Insbesondere hat sich herausgestellt, daß eine derartige Koordination gerade für Kinder sehr schwierig ist. Studien haben gezeigt, daß das erzeugte Aerosol von ungeschickten Benutzern oder beispielsweise Kindern oftmals nicht optimal inhaliert wird.

Die EP 1 163 921 B1 offenbart eine Inhalationsvorrichtung zur Zwischenspeicherung einer zerstäubten Arzneimittelzubereitung (eines Aerosols) in einem beim Inhalieren kollabierenden Behältnis. Die bekannte Inhalationsvorrichtung weist ein vorzugsweise durchsichtiges Gehäuse auf, in dem das Behältnis angeordnet ist. Das Behältnis ist vorzugsweise als im wesentlichen länglicher bzw, zylindrischer Faltenbalg ausgebildet, der sich beim Inhalieren in axialer Richtung bzw. Längsrichtung zusammenzieht. Die bekannte Inhalationsvorrichtung weist eine Steuereinrichtung auf, um den Inhalationsfluß während der gesamten Inhalation des Aerosols im wesentlichen konstant zu halten. Versuche haben gezeigt, daß der Feinpartikelanteil der bekannten Inhalationsvorrichtung nicht optimal ist.

Die DE 20018588 U1 offenbart eine Vorrichtung zur Aufnahme und Abgabe eines Aerosols zu Inhalationszwecken, bei der eine beutelförmige Aufnahmeeinrichtung über eine Zugangsöffnung mit Aerosol befüllbar ist. Die Aufnahmeeinrichtung enthält eine Auffalteinrichtung, durch die die Aufnahmeeinrichtung aus einem vorbestimmten Einfaltzustand in einen stabilen Auffaltzustand bringbar ist.

Die WO2008/124666 A2 offenbart einen so genannten Spacer, d.h. eine Rückhaltekammer zur Anwendung als Inhalationshilfe in Verbindung mit Aerosol abgebenden Systemen. Der Spacer hat ein expandierbares Volumen. Insbesondere werden zusammenfaltbare Spacer zur einmaligen Verwendung offenbart.

Die DE 3513628 C1 offenbart eine Vorrichtung zur Inhalation von Allergenen, in der ein Nebelsammelbehälter an den Nebelauslass eines beispielsweise druckluftbetriebenen Verneblers angeschlossen ist. Nach Ablauf der Vernebelung wird eine Verbindung des Nebelsammelbehälters zu einem Mundstück geöffnet, wodurch die im Behälter befindliche Nebelmenge von einem Patienten eingeatmet werden kann. Der Nebelsammelbehälter besteht aus einem Faltbeutel, der beim Inhalieren seines Inhalts in sich zusammenfallen bzw. sich zusammenfalten oder zusammengedrückt werden kann.

Die WO03/068299 A1 offenbart ein Inhalationshilfsgerät zur Verwendung mit einem Aerosolabgabegerät. Das Inhalationshilfsgerät umfasst eine Kammer, die eine Öffnung zur Verbindung mit dem Aerosolabgabegerät und eine Öffnung mit einem Mundstück aufweist. Die Kammer hat ein veränderbares Volumen und wird von einem sich konisch verjüngenden Faltenbalg aus elastischem Material begrenzt, wobei dieser Faltenbalg in Richtung seiner Längachse zusammengefaltet werden kann.

Der vorliegenden Erfindung liegt die Aufgabe zugrunde, eine Inhalationsvorrichtung zur Zwischenspeicherung einer zerstäubten Arzneimittelzubereitung bzw. eines Aerosols anzugeben, wobei die Inhalationsvorrichtung einen gegenüber dem Stand der Technik höheren Feinpartikelanteil ermöglicht und/oder sehr einfach bzw. kostengünstig aufgebaut ist.

Die obige Aufgabe wird durch eine Inhalationsvorrichtung gemäß Anspruch 1 gelöst. Vorteilhafte Weiterbildungen sind Gegenstand der Unteransprüche.

Ein erster Aspekt der vorliegenden Erfindung liegt darin, daß das beim Inhalieren kollabierende Behältnis derart ausgebildet ist, daß es beim Kollabieren seine Länge zumindest im wesentlichen beibehält. Hierdurch kann das Behältnis leichter kollabieren und/oder können günstigere Strömungsverhältnisse im Behältnis beim Inhalieren bzw. Kollabieren und/oder beim Füllen des Behältnisses mit einer zerstäubten Arzneimittelzubereitung bzw. Aerosol erreicht werden. Dies ist einem höheren Feinpartikelanteil beim Inhalieren zuträglich.

Ein zweiter Aspekt der vorliegenden Erfindung liegt darin, daß sich das Behältnis zu einem freien Ende hin verjüngt. Insbesondere ist das Behältnis an einem Ende an eine Anschlußeinrichtung der Inhalationsvorrichtung angeschlossen oder anschließbar und an seinem anderen (freien) Ende zumindest im wesentlichen flach ausgebildet. Hierdurch kann das Behältnis leichter kollabieren und/oder können günstigere Strömungsverhältnisse im Behältnis beim Inhalieren bzw. Kollabieren und/oder beim Füllen des Behältnisses mit einer zerstäubten Arzneimittelzubereitung bzw. Aerosol erreicht werden. Dies ist einem höheren Feinpartikelanteil beim Inhalieren zuträglich.

Ein dritter Aspekt der vorliegenden Erfindung liegt darin, daß das Behältnis vorzugsweise nicht selbst expandierend ausgebildet ist. Hierdurch kann das Behältnis leichter kollabieren und/oder können günstigere Strömungsverhältnisse im Behältnis beim Inhalieren bzw. Kollabieren und/oder beim Füllen des Behältnisses mit einer zerstäubten Arzneimittelzubereitung bzw. Aerosol erreicht werden. Dies ist einem höheren Feinpartikelanteil beim Inhalieren zuträglich.

Ein vierter Aspekt der vorliegenden Erfindung liegt darin, daß das Behältnis an seinem Entnahmeende den größten Durchmesser oder Querschnitt aufweist, insbesondere zu seinem Entnahmeende hin im Durchmesser oder Querschnitt zunimmt. Hierdurch kann das Behältnis leichter kollabieren und/oder können günstigere Strömungsverhältnisse im Behältnis beim Inhalieren bzw. Kollabieren und/oder beim Füllen des Behältnisses mit einer zerstäubten Arzneimittelzubereitung bzw. Aerosol erreicht werden. Dies ist einem höheren Feinpartikelanteil beim Inhalieren zuträglich.

Ein fünfter Aspekt der vorliegenden Erfindung liegt darin, die Inhalationsvorrichtung lediglich aus einer sich vorzugsweise trichterförmig erweiternden Anschlußeinrichtung und einem daran angeschlossenem oder anschließbaren Beutel als Behältnis aufzubauen, insbesondere also kein Gehäuse oder dergleichen vorzusehen. Die Anschlußeinrichtung bildet dann ein Mundstück und auch eine Halterung für das Behältnis. Hierdurch kann das Behältnis leichter kollabieren und/oder können günstigere Strömungsverhältnisse im Behältnis beim Inhalieren bzw. Kollabieren und/oder beim Füllen des Behältnisses mit einer zerstäubten Arzneimittelzubereitung bzw. Aerosol erreicht werden. Dies ist einem höheren Feinpartikelanteil beim Inhalieren zuträglich.

Ein sechster Aspekt der vorliegenden Erfindung liegt darin, daß die Inhalationsvorrichtung einen sich trichterförmig erweiternden Anschlußbereich für das Behältnis aufweist, insbesondere mit einem Durchmesser, so daß handelsübliche Beutel, Frühstückstüten oder dergleichen als Behältnis anschließbar sind. Hierdurch kann das Behältnis leichter kollabieren und/oder können günstigere Strömungsverhältnisse im Behältnis beim Inhalieren bzw. Kollabieren und/oder beim Füllen des Behältnisses mit einer zerstäubten Arzneimittelzubereitung bzw. Aerosol erreicht werden. Dies ist einem höheren Feinpartikelanteil beim Inhalieren zuträglich.

Die vorgenannten Aspekte der vorliegenden Erfindung und/oder weitere nachfolgend erläuterte Aspekte und Merkmale der vorliegenden Erfindung können unabhängig voneinander und in beliebiger Kombination realisiert werden.

Die vorschlagsgemäße Lösung gestattet insbesondere eine besser definierte Wirkstoffinhalation mit einem letztendlich im Mittel höheren und/oder geringer schwankenden Wirkstoffanteil, der in der Lunge abgeschieden wird. Dies gestattet eine verbesserte Therapie von Kindern und/oder eine Erweiterung der Indikation bzw. den Einsatz von anderen Arzneimittelzubereitungen.

Weitere Vorteile, Merkmale, Eigenschaften und Aspekte der vorliegenden Erfindung ergeben sich aus den Ansprüchen und der folgenden Beschreibung bevorzugter Ausführungsformen anhand der Zeichnung. Es zeigt:
- Fig. 1: eine schematische Schnittdarstellung einer Inhalationsvorrichtung gemäß einer ersten Ausführungsform mit einem zugeordneten Inhalator;
- Fig. 2: eine schematische Darstellung einer vorschlagsgemäßen Inhalationsvorrichtung gemäß einer zweiten Ausführungsform;
- Fig. 3: eine schematische Ansicht eines Behältnisses der Inhalationsvorrichtung; und
- Fig. 4: ein schematisches Schnittmuster zur Herstellung des Behältnisses;
- Fig. 5: einen schematischen Schnitt des Inhalators gemäß einer bevorzugten Ausführungsform im ungespannten Zustand; und
- Fig. 6: einen schematischen, um 90° gegenüber Fig. 5 gedrehten Schnitt des Inhalators im gespannten Zustand.

In den Figuren werden für gleiche oder ähnliche Teile dieselben Bezugszeichen verwendet, wobei entsprechende oder vergleichbare Eigenschaften und Vorteile erreicht werden, auch wenn eine wiederholte Beschreibung weggelassen ist.

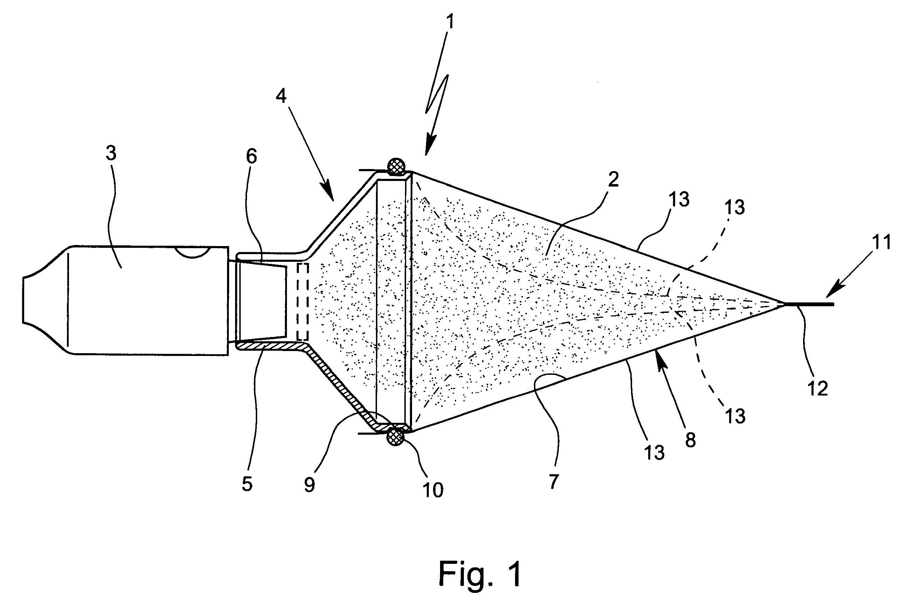

Fig. 1 zeigt in einer schematischen, schnittartigen Darstellung eine Inhalationsvorrichtung 1 gemäß einer ersten Ausführungsform der vorliegenden Erfindung. Die Inhalationsvorrichtung 1 dient einer Zwischenspeicherung eines Aerosols 2. Bei dem Aerosol 2 handelt es sich insbesondere um eine zerstäubte Arzneimittelzubereitung, die inhaliert werden soll.

Das Aerosol 2 wird vorzugsweise von einem Aerosolspender, hier einem Inhalator 3, einem Zerstäuber oder dgl., erzeugt bzw. bereit gestellt und in die Inhalationsvorrichtung 1 abgegeben bzw. dosiert. Besonders bevorzugt handelt es sich bei dem Aerosol 2 um eine treibgasfrei zerstäubte Arzneimittelzubereitung oder dergleichen. Jedoch kann das Aerosol 2 auch durch eine treibgasgetriebene Zerstäubung oder durch treibgasunterstützte Zerstäubung erzeugt werden.

Die Inhalationsvorrichtung 1 ist vorzugsweise temporär mit dem Aerosolspender bzw. Inhalator 3 oder dergleichen fluidisch und vorzugsweise auch mechanisch verbindbar. Fig. 1 zeigt die Inhalationsvorrichtung 1 in einem an den Inhalator 3 angeschlossenen Zustand und in einem bereits mit dem Aerosol 2 gefüllten Zustand.

Die Inhalationsvorrichtung 1 weist eine Anschlußeinrichtung 4 auf, um die Inhalationsvorrichtung 1 zum Füllen bzw. zur Aufnahme des Aerosols 2 fluidisch und vorzugsweise auch mechanisch - beim Darstellungsbeispiel an den Inhalator 3 oder einen sonstigen Aerosolspender oder Zerstäuber oder dergleichen - anzuschließen und/oder um das aufgenommene bzw. zwischengespeicherte Aerosol 2 wieder abzugeben, insbesondere an einen nicht dargestellten Benutzer oder Patienten beim Inhalieren.

Beim Darstellungsbeispiel weist die Anschlußeinrichtung 4 einen vorzugsweise stutzenartigen Anschlußabschnitt 5 zur Aufnahme und/oder Abgabe des Fluids auf. Der Anschlußabschnitt 5 ist beim Darstellungsbeispiel vorzugsweise auf ein Mundstück 6 des Inhalators 3 aufsteckbar oder darin einsteckbar. Insbesondere ist die Inhalationsvorrichtung 1 bzw. deren Anschlußeinrichtung 4 oder deren Anschlußabschnitt 5 klemmend oder rastend mit dem Inhalator 3 lösbar verbindbar. Jedoch sind auch andere konstruktive Lösungen möglich.

Es ist anzumerken, daß beim Darstellungsbeispiel die Verbindungseinrichtung 4 vorzugsweise nur einen kombinierten Ein- und Auslaß für das Aerosol 2 - hier in Form des Anschlußabschnitts 5 - aufweist. Jedoch können der Einund Ausfluß auch separat ausgebildet sein.

Der Anschlußabschnitt 5 ist innen und/oder außen vorzugsweise rund oder oval ausgebildet.

Das Aerosol 2 wird von der Inhalationsvorrichtung 1 zur Zwischenspeicherung in einem Aufnahmeraum 7 aufgenommen.

Die Inhalationsvorrichtung 1 weist ein Behältnis 8 zur Aufnahme bzw. Zwischenspeicherung des Aerosols 2 bzw. zur Bildung (zumindest eines größten Teils) des Aufnahmeraums 7 auf.

Das Behältnis 8 ist vorzugsweise derart ausgebildet, daß es beim Inhalieren - also bei der Entnahme des Aerosols 2 von der Inhalationsvorrichtung 1 bzw. aus dem Aufnahmeraum 7 bzw. aus dem Behältnis 8 - kollabiert.

Das Behältnis 8 ist vorzugsweise aus einem flexiblen und/oder weichen und/oder verformbaren Material, insbesondere aus Papier, Folie, einem geeigneten Verbundmaterial oder dergleichen hergestellt.

Das Behältnis 8 ist vorzugsweise beutelartig ausgebildet.

Das Behältnis 8 ist an die Anschlußeinrichtung 4 bzw. deren Anschlußabschnitt 5 fluidisch und vorzugsweise auch mechanisch angeschlossen oder anschließbar. Beim Darstellungsbeispiel wird das Behältnis 8 von der Anschlußeinrichtung 4 gehalten. Vorzugsweise weist die Anschlußeinrichtung 4 einen Verbindungsabschnitt 9 zur Verbindung mit dem Behältnis 8 auf.

Das Behältnis 8 ist vorzugsweise lösbar, wechselbar und/oder klemmend mit der Anschlußeinrichtung 4 bzw. dem Verbindungsabschnitt 9 verbunden oder verbindbar, ganz besonders bevorzugt beim Darstellungsbeispiel mittels eines Rings oder Befestigungsmittels 10, wie eines Gummibands, O-Rings, Klemmrings oder dergleichen, das beispielsweise das Behältnis 8 klemmend und/oder formschlüssig an dem vorzugsweise stutzenartigen Verbindungsabschnitt 9 bzw. einem Außenumfang des Verbindungsabschnitt 9 hält. Jedoch sind auch andere konstruktive Lösungen möglich. Beispielsweise kann das Behältnis 8 einen verstärkten Rand oder Anschlußbereich aufweisen, der klemmend und/oder auf sonstige Art und Weise mit der Anschlußeinrichtung 4 bzw. dem Verbindungsabschnitt 9 verbindbar ist.

Das Behältnis 8 ist vorzugsweise an einem Ende 11 mit der Anschlußeinrichtung 4 bzw. deren Verbindungsabschnitt 9 verbunden oder verbindbar. Am anderen (freien) Ende 11 ist das Behältnis 8 geschlossen.

Das Behältnis 8 ist an seinem freien Ende 11 besonders bevorzugt mit einer Siegel- oder Schweißnaht 12 geschlossen.

Das Behältnis 8 ist vorzugsweise länglich ausgebildet. Das Behältnis 8 ist vorzugsweise derart ausgebildet, daß es beim Kollabieren seine Länge zumindest im wesentlichen beibehält.

Das Behältnis 8 ist vorzugsweise zu seinem freien Ende 11 hin verjüngt ausgebildet.

Das Behältnis 8 ist zumindest bereichsweise, insbesondere an seinem freien Ende 11 flach ausgebildet.

Das Behältnis 8 weist einen vorzugsweise vom freien Ende 11 aus zur Anschlußeinrichtung 4 hin zunehmenden Durchmesser und/oder Querschnitt auf.

Der Durchmesser oder Querschnitt des Behältnisses 8 ist vorzugsweise im Bereich des Anschlusses an die Anschlußeinrichtung 4 bzw. des Verbindungsabschnitts 9 am größten.

Das Behältnis 8 ist vorzugsweise zumindest im wesentlichen keilförmig ausgebildet.

Das Behältnis 8 weist vorzugsweise zwei gegenüberliegende Flachseiten 13 auf, die beim Inhalieren - also bei der Abgabe des Aerosols 2 aus der Inhalationsvorrichtung 1 - sich aufeinanderzubewegen bzw. zusammenfallen oder zusammengezogen werden, wenn das Behältnis 8 kollabiert.

Fig. 1 zeigt gestrichelt angedeutet das Behältnis 8 im kollabierten Zustand, also nach der Entnahme (eines wesentlichen Teils) des Aerosols 2. Insbesondere sind die Flachseiten 13 zusammengefallen. Die Flachseiten 13 können dabei auch gebogen werden, sich insbesondere nach innen wölben. Weiter kann sich die axiale Länge bzw. die Länge des Behältnisses 8 in Ein- und Ausströmrichtung des Aerosols 2 etwas verringern. Dies wird jedoch nicht als eine wesentliche Änderung der Länge des Behältnisses 8 bei der vorliegenden Erfindung angesehen. Vielmehr wird dies so verstanden, daß das Behältnis 8 seine Länge zumindest im wesentlichen beim Kollabieren beibehält.

Das Kollabieren des Behältnisses 8 erfolgt insbesondere nur durch Heraussaugen des Aerosols 2 aus dem Aufnahmeraum 7. Das Behältnis 8 soll dementsprechend möglichst leicht kollabieren, um das Aerosol 2 mit möglichst geringem Druckverlust entnehmen zu können.

Das vorschlagsgemäße Behältnis 8 kollabiert aufgrund seiner vorgesehenen Form besonders leicht. Dies unterstützt die Ausgabe des Aerosols 2 mit einem besonders hohen Feinpartikelanteil.

Aufgrund der keilförmigen Form bzw. aufgrund des zum freien Ende 11 hin vorzugsweise kontinuierlich abnehmenden Durchmessers und/oder Querschnitts des Behältnisses 8 ist der mittlere Weg, den das Aerosol 2 bei der Aufnahme und Entnahme insgesamt zurücklegen muß gegenüber einem im wesentlichen über die gesamte Länge konstanten Querschnitt des Behältnisses 8 verringert.

Das Behältnis 8 kann im Bereich der Verbindung mit der Anschlußeinrichtung 4 einen im wesentlich rechteckigen oder runden, insbesondere ovalen oder kreisförmigen Querschnitt aufweisen. Jedoch sind hier auch andere Formen möglich.

Es ist anzumerken, dass das Behältnis 8 auch über einen flexiblen Verbindungsbereich 9, dessen Anschlußöffnung zum Behältnis 8 hin ggf. sogar selbst kollabieren kann, an die Anschlußeinrichtung 4 angeschlossen sein kann.

Nachdem die Inhalationsvorrichtung 1 bzw. das Behältnis 8 das Aerosol 2 zur Zwischenspeicherung aufgenommen hat, wird der Aerosolspender bzw. der Inhalator 3 oder dergleichen von der Inhalationsvorrichtung 1 getrennt. Anschließend kann ein nicht dargestellter Benutzer oder Patient das Aerosol 2 der Inhalationsvorrichtung 1 durch Einatmen bzw. Inhalieren entnehmen. Hierzu weist die Inhalationsvorrichtung 1 bzw. Anschlußeinrichtung 4 eine entsprechende Entnahmemöglichkeit auf. Diese Entnahmemöglichkeit wird besonders bevorzugt durch den Anschlußabschnitt 5 gebildet, der beispielsweise hierzu mit einem nicht dargestelltem Mundstück o. dgl. verbindbar ist und/oder selbst ein Mundstück für den Benutzer bzw. Patienten bildet. Besonders bevorzugt kann der Benutzer bzw. Patient also den Anschlußabschnitt 5 direkt in den Mund nehmen und das Aerosol 2 durch Einatmen der Inhalationsvorrichtung 1 bzw. dem Behältnis 8 entnehmen, also das Aerosol 2 heraussaugen.

Die Inhalationsvorrichtung 1 ist beim Darstellungsbeispiel besonders bevorzugt lediglich aus der Anschlußeinrichtung 4 und dem Behältnis 8 sowie gegebenenfalls dem Befestigungsmittel 10 (sofern dies erforderlich ist bzw. kein Teil der Anschlußeinrichtung 4 oder des Behältnisses 8 bildet) gebildet.

Die Anschlußeinrichtung 4 ist vorzugsweise mehrfach verwendbar ausgebildet und insbesondere sehr einfach aufgebaut und zu reinigen, insbesondere spülmaschinenfest, und kann gegebenenfalls durch Abkochen gereinigt werden.

Beim Darstellungsbeispiel ist die Anschlußeinrichtung 4 mit dem Anschlußabschnitt 5 und dem Verbindungsabschnitt 9 vorzugsweise einstückig, insbesondere als Spritzgußteil ausgebildet. Die Anschlußeinrichtung 4 kann jedoch auch mehrteilig ausgebildet bzw. aus mehreren Komponenten lösbar oder unlösbar zusammengebaut sein.

Die Anschlußeinrichtung 4 besteht vorzugsweise aus einem geeigneten Kunststoff.

Die Inhalationsvorrichtung 1 bzw. die Anschlußeinrichtung 4 erweitert sich vorzugsweise von dem Anschlußabschnitt 5 zum Behältnis 8 bzw. zum Verbindungsabschnitt 9 hin, insbesondere trichterförmig. Jedoch sind auch andere konstruktive Lösungen möglich.

Das Behältnis 8 ist vorzugsweise nur einmalig verwendbar bzw. als Einwegartikel ausgebildet, beispielsweise kann es sich hierbei um handelsübliche Beutel oder Tüten, wie Frühstückstüten oder dergleichen handeln.

Die Inhalationsvorrichtung 1 bzw. die Anschlußeinrichtung 4 kann optional eine in Fig. 1 nur gestrichelt angedeutete Ventil- bzw. Steuereinrichtung 14 aufweisen. Diese Ventil- bzw. Steuereinrichtung 14 kann beispielsweise den Ein- und/oder Ausströmungswiderstand oder die Ein- und/oder Ausströmrate steuern oder regeln und/oder ein temporäres Verschließen ermöglichen.

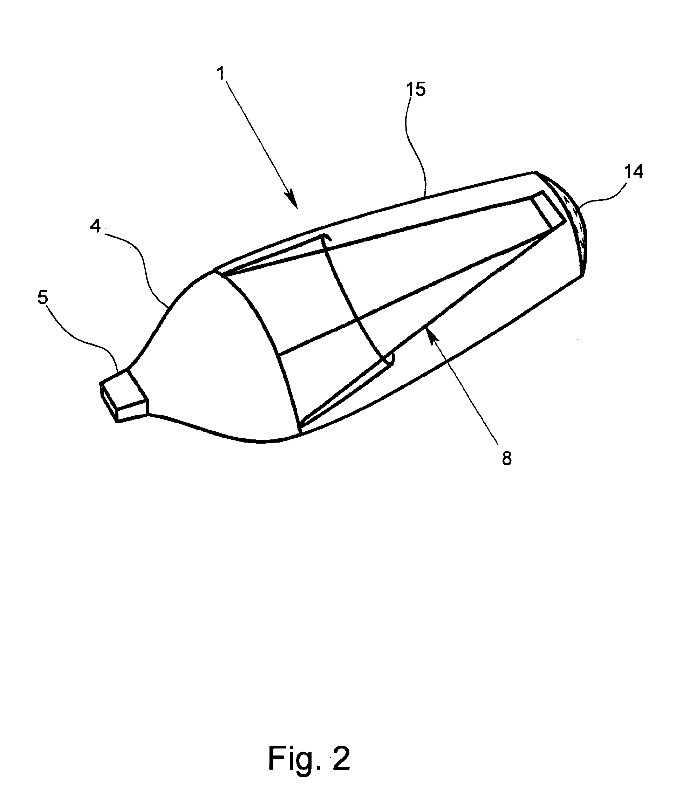

Nachfolgend wird anhand der Fig. 2 bis 4 eine zweite Ausführungsform der vorschlagsgemäßen Inhalationsvorrichtung 1 erläutert, wobei die folgende Beschreibung auf wesentliche Unterschiede und/oder zusätzliche Aspekte fokussiert. Die bisherigen Ausführungen und Erläuterungen gelten insbesondere ergänzend oder entsprechend. Des weiteren können die beiden Ausführungsformen bzw. einzelne Aspekte und Merkmale der Ausführungsformen auch beliebig miteinander kombiniert, aber auch unabhängig voneinander und gegebenenfalls auch bei sonstigen anderen Inhalationsvorrichtungen realisiert werden.

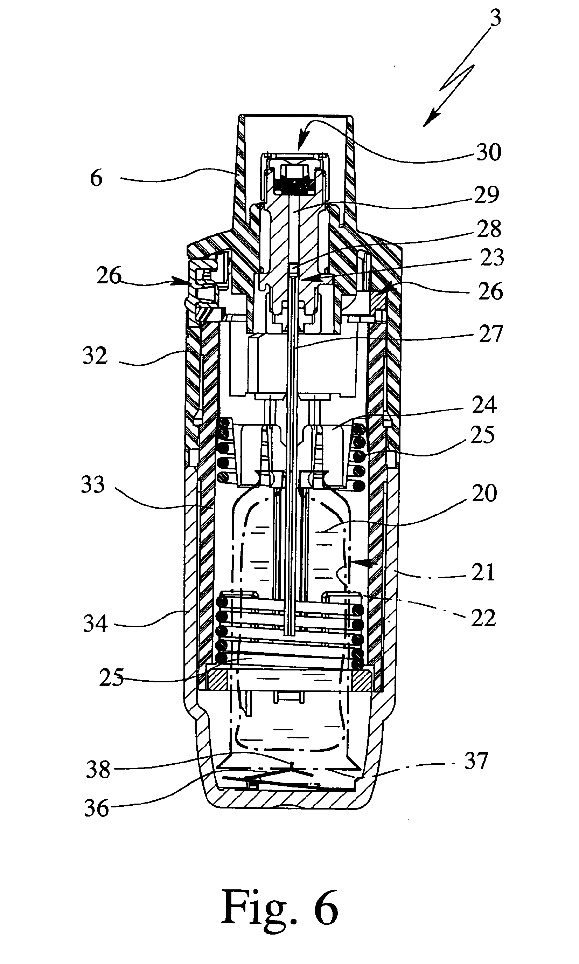

Fig. 2 zeigt in einer schematischen Darstellung die Inhalationsvorrichtung 1 gemäß der zweiten Ausführungsform. Diese Ausführungsform entspricht vorzugsweise im wesentlichen der in der EP 1 163 921 B1 in Fig. 6 a oder b beschriebenen Ausführungsform einer Inhalationsvorrichtung.

Im Gegensatz zu dem balgförmigen, in axialer Richtung kollabierendem Behältnis weist die Inhalationsvorrichtung 1 gemäß der zweiten Ausführungsform das Behältnis 8 gemäß der ersten Ausführungsform auf. Das Behältnis 8 ist vorzugsweise also zumindest im wesentlichen keilförmig ausgebildet und/oder derartig ausgebildet, daß es beim Kollabieren seine Länge zumindest im wesentlichen beibehält. Außerdem ist das Behältnis 8 vorzugsweise zu seinem freien Ende 11 hin verjüngt.

Die Inhalationsvorrichtung 1 gemäß der zweiten Ausführungsform weist gegenüber der ersten Ausführungsform insbesondere ein Gehäuse 15 auf, das das Behältnis 8 umgibt. Das Gehäuse 15 ist vorzugsweise zumindest teilweise transparent oder durchsichtig ausgebildet, so daß das Behältnis 8 sichtbar ist, insbesondere um zu sehen bzw. überprüfen zu können, ob das Behältnis 8 expandiert ist, kollabiert ist oder inwieweit es kollabiert ist.

Das Gehäuse 15 ist an einem Ende vorzugsweise lösbar mit der Anschlußeinrichtung 4 verbunden oder verbindbar. Am anderen bzw. freien Ende weist das Gehäuse 15 vorzugsweise die optionale Ventil- bzw. Steuereinrichtung 14 auf.

Bei der zweiten Ausführungsform ist das Behältnis 8 vorzugsweise derart in die Inhalationsvorrichtung 1 bzw. im Gehäuse 15 angeordnet, daß beim Expandieren zur Aufnahme von Aerosol 2 in das Behältnis 8 und/oder beim Kollabieren zur Entnahme von Aerosol 2 aus dem Behältnis 8 Luft aus dem das Behältnis 8 umgebenden Raum aus der Inhalationsvorrichtung 1 bzw. dem Gehäuse 15 heraus oder hinein strömt. Dieser Luftstrom ist vorzugsweise gedrosselt und/oder gesteuert oder geregelt, besonders bevorzugt durch die optionale Ventil- bzw. Steuereinrichtung 14. Jedoch sind hier auch andere konstruktive Lösungen möglich.

Der Verbindungsabschnitt 9 der Anschlußeinrichtung 4 für das Behältnis 8 ist in Fig. 2 nicht sichtbar, sondern beispielsweise von einem äußeren Gehäuseabschnitt der Inhalationsvorrichtung 1 bzw. der Anschlußeinrichtung 4 bzw. dem Gehäuse 15 abgedeckt.

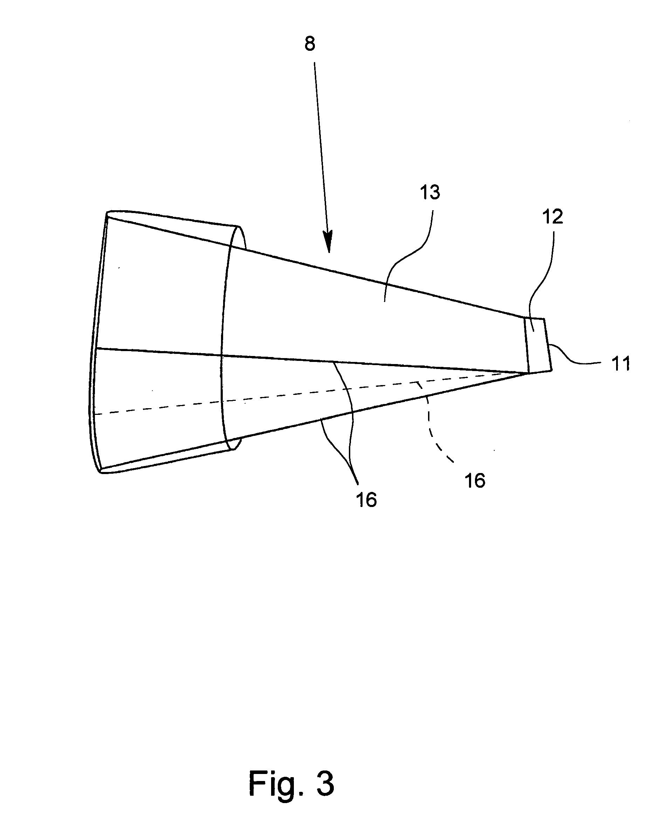

Fig. 3 zeigt in einer perspektivischen Ansicht das Behältnis 8, es weist vorzugsweise mindestens eine Knick-, Falt- oder Biegekante 16, besonders bevorzugt auf beiden Seiten und/oder entlang beider Flachseiten 13, auf, die zumindest im wesentlichen in Längserstreckung und/oder in Haupteinströmrichtung oder Ausströmrichtung des Aerosols 2 verläuft, um ein definiertes und/oder leichtes Kollabieren des Behältnisses 8 bei der Entnahme von Aerosol 2 also beim Inhalieren zu ermöglichen oder gewährleisten.

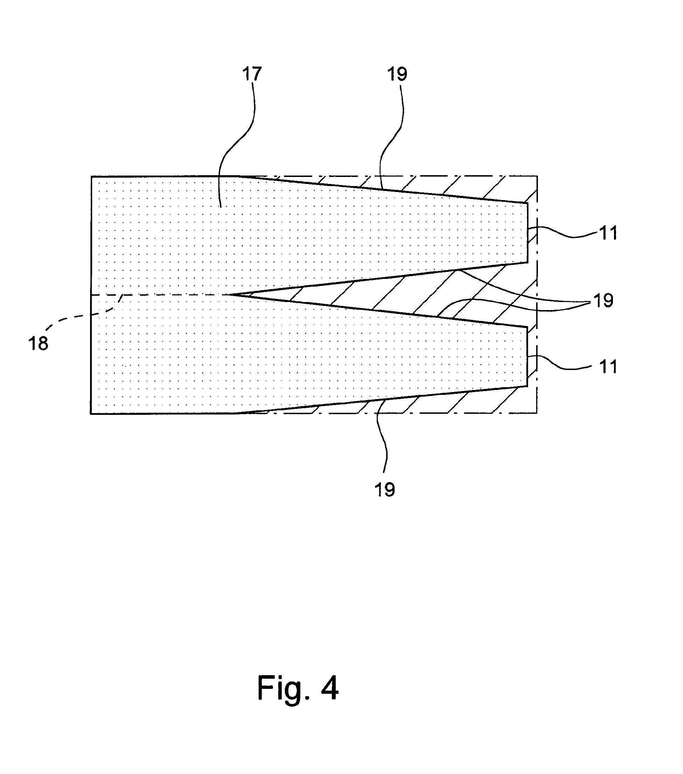

Das Behältnis 8 ist vorzugsweise aus einem Materialstück bzw. Zuschnitt 17 hergestellt, wie in Fig. 4 beispielhaft angedeutet. Der Zuschnitt 17 wird beispielsweise durch Zuschneiden eines größeren Materialstücks - beim Darstellungsbeispiel durch Wegschneiden der groß schraffierten Bereiche - gebildet.

Der Zuschnitt 17 wird vorzugsweise längs gefaltet, hier entlang der Linie 18, und/oder so, daß zwei Materialstücke oder Bereiche aufeinanderzuliegen kommen, die dann an den Längsränder 19 und am freien Ende 11 miteinander verbunden werden, insbesondere durch Kleben, Siegeln und/oder Schweißen.

So wird ein kollabierbarer Beutel als Behältnis 8 gebildet wird. Der Beutel 8 ist an dem dem freien Ende 11 gegenüberliegendem Ende offen ausgebildet und an diesem Ende mit der Inhalationsvorrichtung 1 bzw. der Anschlußeinrichtung 4 bzw. dem Verbindungsabschnitt 9 vorzugsweise lösbar verbindbar.

So kann das Behältnis 8 auf sehr einfache und kostengünstige Weise hergestellt werden.

Versuche mit einer Meßeinrichtung haben gezeigt, dass der Feinpartikelanteil durch die vorschlagsgemäße Formgebung bzw. Modifikation des Behältnisses 8 wesentlich erhöht werden kann. Insbesonders kann der Feinpartikelanteil ausgehend von der bekannten Inhalationsvorrichtung gemäß Fig. 6a der EP 1 163 921 B1 durch die Modifikation des Behältnisses 8 um mehr als 50 % erhöht werden, wie Versuche ergeben haben.

Es ist anzumerken, daß die Inhalationsvorrichtung 1 bzw. das Behältnis 8 vorzugsweise nur ein bestimmtes Aerosolvolumen aufnimmt.

Es ist anzumerken, daß das Behältnis 8 vorzugsweise nicht selbstexpandierend ausgebildet ist.

Nachfolgend wird eine besonders bevorzugte Ausführungsform des Inhalators 3 näher erläutert.

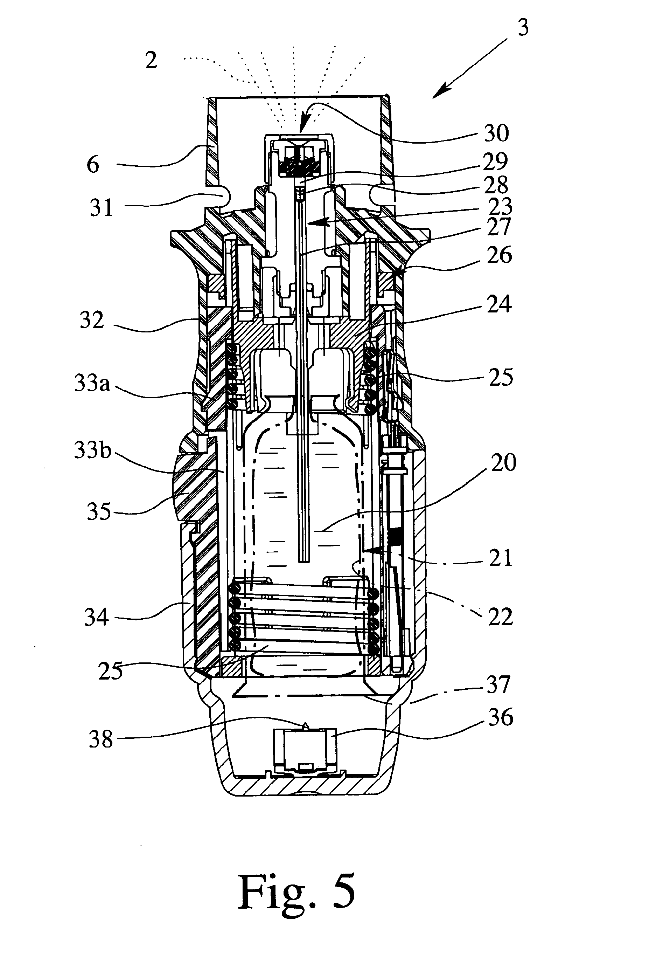

Fig. 5 und 6 zeigen einen vorschlagsgemäßen, tragbaren Inhalator 3 zur treibgasfreien Zerstäubung einer Arzneimittelzubereitung 20 in einer schematischen Darstellung im ungespannten Zustand (Fig. 5) und gespannten Zustand (Fig. 6). Fig. 5 und 6 zeigen den Inhalator 3 mit einem Behälter 21 mit der Arzneimittelzubereitung 20.

Bei Zerstäubung der Arzneimittelzubereitung 20, vorzugsweise einer Flüssigkeit, wird ein lungengängiges Aerosol 2 (Fig. 5) gebildet, das von einem nicht dargestellten Benutzer bzw. Patienten eingeatmet bzw. inhaliert werden kann. Üblicherweise erfolgt das Inhalieren wenigstens einmal täglich, insbesondere mehrmals täglich, vorzugsweise in vorbestimmten Zeitabständen, insbesondere in Abhängigkeit von der Erkrankung des Patienten.

Der Inhalator 3 weist den vorzugsweise einsetzbaren und ggf. wechselbaren Behälter 21 mit der Arzneimittelzubereitung 20 auf. Der Behälter 21 bildet also ein Reservoir für die zu zerstäubende Arzneimittelzubereitung 20. Vorzugsweise enthält der Behälter 21 eine ausreichende Menge an Arzneimittelzubereitung 20 bzw. Wirkstoff für mehrere Dosen des Arzneimittelzubereitung 20, also mehrere Zerstäubungen oder Anwendungen zu ermöglichen. Ein typischer Behälter 21, wie in der WO 96/06011 A1 offenbart, nimmt ein Volumen von ca. 2 bis 10 ml auf. Hinsichtlich des bevorzugten Aufbaus des Behälters 21 wird ergänzend auf die WO 00/49988 A2 verwiesen.

Der Behälter 21 ist vorzugsweise im wesentlichen zylindrisch bzw. kartuschenartig ausgebildet und von unten, nach Öffnen des Inhalators 3, in diesen einsetzbar und ggf. wechselbar. Er ist vorzugsweise starr ausgebildet, insbesondere wobei das Arzneimittelzubereitung 20 in einem kollabierbaren Beutel 22 im Behälter 21 aufgenommen ist.

Der Inhalator 3 weist ferner eine Fördereinrichtung, insbesondere einen Druckerzeuger 23, zur Förderung und Zerstäubung der Arzneimittelzubereitung 20, insbesondere jeweils in einer vorbestimmten, ggf. einstellbaren Dosiermenge auf.

Der Inhalator 3 bzw. Druckerzeuger 23 weist insbesondere eine Halterung 24 für den Behälter 21, eine zugeordnete, nur teilweise dargestellte Antriebsfeder 25 vorzugsweise mit einem zugeordneten, zur Entsperrung manuell betätigbaren Sperrelement 26, ein Förderelement, vorzugsweise ein als Kapillare ausgebildetes Förderrohr 27, mit einem optionalen Ventil, insbesondere Rückschlagventil 28, eine Druckkammer 29 und/oder eine Austragsdüse 30 insbesondere im Bereich eines Mundstücks 6 auf.

Der Behälter 21 wird über die Halterung 24, insbesondere klemmend oder rastend, so in dem Inhalator 1 fixiert, daß das Förderrohr 27 in den Behälter 21 eintaucht. Die Halterung 24 kann dabei derart ausgebildet sein, daß der Behälter 21 ausgetauscht werden kann.

Beim axialen Spannen der Antriebsfeder 25 wird die Halterung 24 mit dem Behälter 21 und dem Förderrohr 27 bei den Darstellungen nach unten bewegt und die Arzneimittelzubereitung 20 - genauer gesagt die nächste Dosis - aus dem Behälter 21 in die Druckkammer 29 des Druckerzeugers 23 über das Rückschlagventil 28 gesaugt.

Beim anschließenden Entspannen nach Betätigung des Sperrelements 26 wird die Arzneimittelzubereitung 20 in der Druckkammer 29 unter Druck gesetzt, indem das Förderrohr 27 bei nun geschlossenem Rückschlagventil 28 durch Entspannen der Antriebsfeder 25 wieder nach oben bewegt wird und nun als Druckstempel wirkt. Dieser Druck treibt die Arzneimittelzubereitung 20 durch die Austragsdüse 30 aus, wobei es in das vorzugsweise lungengängige Aerosol 2 zerstäubt wird, wie in Fig. 5 angedeutet.

Der nicht dargestellte Benutzer bzw. Patient kann das Aerosol 2 inhalieren, wobei vorzugsweise Zuluft über mindestens eine Zuluftöffnung 31 in das Mundstück 6 saugbar ist.

Während des Zerstäubungsvorgangs wird der Behälter 21 von der Antriebsfeder 25 wieder in seine Ausgangslage zurückbewegt. Der Behälter 21 führt also eine Hubbewegung während des Spannvorgangs und während des Zerstäubungsvorgangs aus.

Der Inhalator 3 weist insbesondere ein erstes Gehäuseteil (Oberteil) 32 und ein demgegenüber drehbares Innenteil 33 (Fig. 6) mit einem oberen Teil 33a und einem unteren Teil 33b (Fig. 5) auf, wobei an dem Innenteil 17 ein insbesondere manuell betätigbares oder drehbares, zweites Gehäuseteil (Unterteil) 34 vorzugsweise mittels eines Sicherheitsverschlusses bzw. Halteelements 35 lösbar befestigt, insbesondere aufgesteckt, ist. Insbesondere ist der Sicherheitsverschluß bzw. das Halteelement 35 derart ausgebildet, daß ein versehentliches Öffnen des Inhalators 3 bzw. Abziehen des zweiten Gehäuseteils 34 ausgeschlossen ist. Insbesondere muß zum Lösen des zweiten Gehäuseteils 34 das Halteelement 35 gegen Federkraft eingedrückt werden. Zum Einsetzen und/oder Auswechseln des Behälters 21 ist das zweite Gehäuseteil 34 vom Inhalator 1 lösbar. Das zweite Gehäuseteil 34 bildet vorzugsweise ein kappenartiges Gehäuseunterteil und/oder um- bzw. übergreift einen unteren freien Endbereich des Behälters 21.

Das zweite Gehäuseteil 34 kann relativ zum ersten Gehäuseteil 32 gedreht werden, wobei das Innenteil 33 mitgedreht wird. Dadurch wird die Antriebsfeder 25 über ein nicht im einzelnen dargestelltes, auf die Halterung 24 wirkendes Getriebe in axialer Richtung gespannt. Mit dem Spannen wird der Behälter 21 axial nach unten bzw. mit seinem Endbereich (weiter) in das zweite Gehäuseteil 34 bzw. zu dessen stimseitigem Ende hin bewegt, bis der Behälter 21 eine in Fig. 6 angedeutete Endlage einnimmt. In diesem Zustand ist die Antriebsfeder 25 bzw. der Inhalator 3 gespannt und gesperrt.

Der Inhalator 25 weist vorzugsweise eine Einrichtung zur zwangsweisen Belüftung des Behälters 21 auf.

Beim erstmaligen Spannen erfolgt vorzugsweise ein bodenseitiges Anstechen bzw. Öffnen des Behälters 21. Insbesondere kommt eine axial wirkende, im Gehäuseteil 34 angeordnete Feder 36 am Behälterboden 37 zur Anlage, die mit einem Anstechelement 38 den Behälter 21 bzw. eine bodenseitige, insbesondere gasdichte Versiegelung bei der erstmaligen Anlage zur Belüftung ansticht.

Die Einrichtung zur zwangsweisen Belüftung ist hier also durch das Anstechelement 38 gebildet, das von der Feder 36 gehalten oder gebildet ist. Jedoch sind auch andere konstruktive Lösungen möglich.

Es ist anzumerken, daß bei dem Anstechen zur Belüftung lediglich die Außenhülle des Behälters 21 geöffnet wird. Der Beutel 22 mit der Arzneimittelzubereitung 20 bleibt unbeschädigt. Bei der Entnahme der Arzneimittelformulierung 20 aus dem Beutel 22 über das Förderrohr 27 kollabiert der flexible Beutel 22. Zum Druckausgleich kann die Umgebungsluft über die Belüftungs- bzw. Anstechöffnung in den Behälter 21 nachströmen.

Zur Benutzung des Inhalators 3 muß zunächst der Behälter 21 eingesetzt werden. Dies erfolgt vorzugsweise dadurch, daß das zweite Gehäuseteil 34 entfernt bzw. abgezogen wird. Anschließend wird der Behälter 21 in das Innenteil 33 axial eingeführt bzw. eingeschoben. Hierbei erfolgt ein kopfseitiges Öffnen bzw. Anschließen des Behälters 21. Dies erfolgt durch das Förderelement, also das Förderrohr 27, das eine vorzugsweise vorgesehene, kopfseitige Versiegelung des Behälters 21 durchsticht und anschließend durch ein kopfseitiges Septum des Behälters 21 hindurch in das Innere des Beutels 22 eingeführt wird. So wird die fluidische Verbindung zwischen dem Behälter 21-genauer gesagt zwischen dem Beutel 22 im Behälter 21- über das Förderrohr 27 zum Druckerzeuger 23 bzw. zur Druckkammer 29 hergestellt.

Anschließend wird das zweite Gehäuseteil 34 wieder aufgeschoben. Nun kann das erstmalige Spannen des Inhalators 3 erfolgen. Hierbei wird dann der Behälter 21 durch das Anstechelement 38 bodenseitig angestochen, also zwangsweise belüftet, wir bereits erläutert.

Vor der erstmaligen Benutzung erfolgt nach dem Einsetzen bzw. fluidischen Anschließen des Behälters 21 ein vorzugsweise mehrmaliges Spannen und Auslösen des Inhalators 3. Durch dieses sogenannte Primen wird im Förderrohr 27 und im Druckerzeuger 23 bis zur Austragsdüse 30 eventuell vorhandene Luft von der Arzneimittelzubereitung 20 verdrängt. Anschließend ist der Inhalator 3 zur Inhalation bereit.

Die Menge der pro Hub bzw. Zerstäubungsvorgang ausgebrachten Arzneimittelzubereitung 20 beträgt vorzugsweise etwa 10 µl bis 50 µl, insbesondere etwa 10 µl bis 20 µl, ganz besonders bevorzugt etwa 15 µl.

Die Antriebsfeder 25 ist vorzugsweise vorgespannt eingebaut, um einen hohen Förderdruck zu erreichen. Beim vorschlagsgemäßen Inhalator 3 erfolgt das Unterdrucksetzen und Fördern der Arzneimittelzubereitung 20 während des Zerstäubungsvorgangs nämlich vorzugsweise nur durch Federkraft, insbesondere nur durch die Kraft der Antriebsfeder 25.

Der Inhalator 3 ist vorzugsweise derart ausgebildet, daß die Arzneimittelzubereitung 20 im Druckerzeuger 23 bzw. in der Druckkammer 29 bei der Ausgabe einem Druck von 5 MPa bis 60 MPa, insbesondere etwa 10 MPa bis 50 MPa, erreicht. Besonders bevorzugt wird bei der Ausgabe bzw. Zerstäubung der Arzneimittelzubereitung 20 ein Druck von etwa 5 MPa bis 60 MPa, insbesondere etwa 10 bis 30 MPa, an der Austragsdüse 30 bzw. an deren Düsenöffnungen erreicht. Die Arzneimittelzubereitung 20 wird dann in das Aerosol 2 überführt, dessen Tröpfchen einen aerodynamischen Durchmesser von bis zu 20 µm, bevorzugt etwa 3 µm bis 10 µm, aufweisen. Die Zerstäubungswirkung bzw. der Zerstäubungseffekt wird durch sich vorzugsweise kreuzende Strahlen, die von der Austragsdüse 30 abgegeben werden, bewirkt bzw. weiter unterstützt.

Der Inhalator 3 ist vorzugsweise derart ausgebildet, daß das Aerosol 2 mit geringer Geschwindigkeit, insbesondere mit einer Geschwindigkeit von weniger als 2 m/s, besonders bevorzugt von etwa 1,6 m/s oder weniger, ausgegeben wird (jeweils gemessen in 10 cm Abstand von der Austragsdüse 30). Der Inhalator 3 ist also vorzugsweise als sogenannter SMI (soft mist inhaler) ausgebildet. Die geringe Ausgabegeschwindigkeit kann insbesondere durch sich kreuzende Strahlen der Arzneimittelzubereitung 20, die von der Austragsdüse 30 abgegeben werden, und/oder entsprechende Wahl der Federkraft bewirkt bzw. unterstützt werden.

Besonders bevorzugt ist der Inhalator 3 derart ausgebildet, daß die Aerosolerzeugung jeweils über mindestens 1 s, insbesondere über mindestens 1,5 s, dauert. Die Zeitdauer zur Zerstäubung einer Dosis bzw. bei einer Betätigung des Inhalators 1 beträgt also mindestens 1 s, insbesondere über 1,5 s.

Zur Vervollständigung der Offenbarung der vorliegenden Patentanmeldung und zur bevorzugten Ausbildung des Inhalators 3 wird vorsorglich auf den kompletten Offenbarungsgehalt sowohl der WO 91/14468 A1 als auch der WO 97/12687 A1 verwiesen.

Im Gegensatz zu Standgeräten oder dergleichen ist der vorschlagsgemäße Inhalator 3 vorzugsweise transportabel ausgebildet, insbesondere handelt es sich um ein mobiles Handgerät.

Aufgrund seiner zylinderähnlichen Form und einer handlichen Größe von weniger als 9 bis 15 cm in der Länge und 2 bis 4 cm in der Breite kann der Inhalator 3 jederzeit vom Patienten mitgeführt werden. Der Vernebler versprüht ein definiertes Volumen der Arzneimittelzubereitung 20 unter Anwendung hoher Drücke durch kleine Düsen, so daß inhalierbare Aerosole 2 entstehen.

Der vorschlagsgemäße Inhalator 3 arbeitet insbesondere rein mechanisch. Jedoch kann der Inhalator 3 grundsätzlich auch auf jede sonstige Art und Weise arbeiten. Insbesondere ist der Begriff "Fördereinrichtung" bzw. "Druckerzeuger" sehr allgemein zu verstehen. Beispielsweise kann der zur Ausgabe und Zerstäubung erforderliche Druck auch durch Treibgas, eine Pumpe oder auf jede sonstige geeignete Art und Weise erzeugt werden.

Der vorschlagsgemäße Inhalator 3 ist insbesondere zur kurzzeitigen Zerstäubung der Arzneimittelzubereitung 20, beispielsweise für ein bis zwei Atemzüge, ausgebildet. Jedoch kann er auch zur längeren oder kontinuierlichen Zerstäubung ausgebildet bzw. einsetzbar sein.

Nachfolgend werden bevorzugte Verbindungen, Bestandteile und/oder Formulierungen der Arzneimittelzubereitung 20 aufgeführt.

Die unten genannten Verbindungen können allein oder in Kombination zur Anwendung in der erfindungsgemäßen Vorrichtung gelangen. In den unten genannten Verbindungen ist W einen pharmakologisch, aktiver Wirkstoff und (beispielsweise) ausgewählt aus der Gruppe bestehend aus Betamimetika, Anticholinergika, Corticosteroiden, PDE4-Inhibitoren, LTD4-Antagonisten, EGFR-Hemmern, Dopamin-Agonisten, H1-Antihistaminika, PAF-Antagonisten und PI3-Kinase Inhibitoren. Weiterhin können zwei- oder dreifach Kombinationen von W kombiniert werden und zur Anwendung in der erfindungsgemäßen Vorrichtung gelangen. Beispielhaft genannte Kombinationen von W wären:
- W stellt ein Betamimetika dar, kombiniert mit einem Anticholinergika, Corticosteroide, PDE4-Inhibitore, EGFR-Hemmern oder LTD4-Antagonisten,
- W stellt ein Anticholinergika dar, kombiniert mit einem Betamimetika, Corticosteroiden, PDE4-Inhibitoren, EGFR-Hemmern oder LTD4-Antagonisten,
- W stellt ein Corticosteroiden dar, kombiniert mit einem PDE4-Inhibitoren, EGFR-Hemmern oder LTD4-Antagonisten
- W stellt ein PDE4-Inhibitoren dar, kombiniert mit einem EGFR-Hemmern oder LTD4-Antagonisten
- W stellt ein EGFR-Hemmern dar, kombiniert mit einem LTD4-Antagonisten.

Als Betamimetika gelangen hierbei vorzugsweise Verbindungen zur Anwendung, die ausgewählt sind aus der Gruppe bestehend aus Albuterol, Arformoterol, Bambuterol, Bitolterol, Broxaterol, Carbuterol, Clenbuterol, Fenoterol, Formoterol, Hexoprenaline, Ibuterol, Isoetharine, Isoprenaline, Levosalbutamol, Mabuterol, Meluadrine, Metaproterenol, Orciprenaline, Pirbuterol, Procaterol, Reproterol, Rimiterol, Ritodrine, Salmefamol, Salmeterol, Soterenol,

Sulphonterol, Terbutaline, Tiaramide, Tolubuterol, Zinterol, CHF-1035, HO-KU-81, KUL-1248 und
- 3-(4-{6-[2-Hydroxy-2-(4-hydroxy-3-hydroxymethyl-phenyl)-ethylamino]-hexyloxy}-butyl)-benzyl-sulfonamid
- 5-[2-(5,6-Diethyl-indan-2-ylamino)-1-hydroxy-ethyl]-8-hydroxy-1H-quinolin-2-on
- 4-Hydroxy-7-[2-{[2-{[3-(2-phenylethoxy)propyl]sulphonyl}ethyl]-amino}ethyl]-2(3H)-benzothiazolon
- 1-(2-Fluor-4-hydroxyphenyl)-2-[4-(1-benzimidazolyl)-2-methyl-2-butylamino]ethanol
- 1-[3-(4-Methoxybenzyl-amino)-4-hydroxyphenyl]-2-[4-(1-benzimidazolyl)-2-methyl-2-butylamino]ethanol
- 1-[2H-5-hydroxy-3-oxo-4H-1,4-benzoxazin-8-yl]-2-[3-(4-N,N-dimethylaminophenyl)-2-methyl-2-propylamino]ethanol
- 1-[2H-5-hydroxy-3-oxo-4H-1,4-benzoxazin-8-yl]-2-[3-(4-methoxyphenyl)-2-methyl-2-propylamino]ethanol
- 1-[2H-5-hydroxy-3-oxo-4H-1,4-benzoxazin-8-yl]-2-[3-(4-n-butyloxyphenyl)-2-methyl-2-propylamino]ethanol
- 1-[2H-5-hydroxy-3-oxo-4H-1,4-benzoxazin-8-yl]-2-{4-[3-(4-methoxyphenyl)-1,2,4-triazol-3-yl]-2-methyl-2-butylamino}ethanol
- 5-Hydroxy-8-(1-hydroxy-2-isopropylaminobutyl)-2H-1,4-benzoxazin-3-(4H)-on
- 1-(4-Amino-3-chlor-5-trifluormethylphenyl)-2-tert.-butylamino)ethanol
- 6-Hydroxy-8-{1-hydroxy-2-[2-(4-methoxy-phenyl)-1,1-dimethyl-ethylamino]-ethyl}-4H-benzo[1,4]oxazin-3-on
- 6-Hydroxy-8-{1-hydroxy-2-[2-(4-phenoxy-essigsäureethylester)-1,1-dimethyl-ethylamino]-ethyl}-4H-benzo[1,4]oxazin-3-on
- 6-Hydroxy-8-{1-hydroxy-2-[2-(4-phenoxy-essigsäure)-1,1-dimethyl-ethylamino]-ethyl}-4H-benzo[1,4]oxazin-3-on
- 8-{2-[1,1-Dimethyl-2-(2,4,6-trimethylphenyl)-ethylamino]-1-hydroxyethyl}-6-hydroxy-4H-benzo[1,4]oxazin-3-on
- 6-Hydroxy-8-{1-hydroxy-2-[2-(4-hydroxy-phenyl)-1,1-dimethyl-ethylamino]-ethyl}-4H-benzo[1,4]oxazin-3-on
- 6-Hydroxy-8-{1-hydroxy-2-[2-(4-isopropyl-phenyl)-1,1dimethyl-ethylamino]-ethyl}-4H-benzo[1,4]oxazin-3-on
- 8-{2-[2-(4-Ethyl-phenyl)-1,1-dimethyl-ethylamino]-1-hydroxy-ethyl}-6-hydroxy-4H-benzo[1,4]oxazin-3-on
- 8-{2-[2-(4-Ethoxy-phenyl)-1,1-dimethyl-ethylamino]-1-hydroxy-ethyl}-6-hydroxy-4H-benzo[1,4]oxazin-3-on
- 4-(4-{2-[2-Hydroxy-2-(6-hydroxy-3-oxo-3,4-dihydro-2H-benzo[1,4]oxazin-8-yl)-ethylamino]-2-methyl-propyl}-phenoxy)-buttersäure
- 8-{2-[2-(3,4-Difluor-phenyl)-1,1-dimethyl-ethylamino]-1-hydroxy-ethyl}-6-hydroxy-4H-benzo[1,4]oxazin-3-on
- 1-(4-Ethoxy-carbonylamino-3-cyano-5-fluorophenyl)-2-(tert.-butylamino)ethanol
- 2-Hydroxy-5-(1-hydroxy-2-{2-[4-(2-hydroxy-2-phenyl-ethylamino)-phenyl]-ethylamino}-ethyl)-benzaldehyd
- N-[2-Hydroxy-5-(1-hydroxy-2-{2-[4-(2-hydroxy-2-phenyl-ethylamino)-phenyl]-ethylamino}-ethyl)-phenyl]-formamid
- 8-Hydroxy-5-(1-hydroxy-2-{2-[4-(6-methoxy-biphenyl-3-ylamino)-phenyl]-ethylamino}-ethyl)-1H-quinolin-2-on
- 8-Hydroxy-5-[1-hydroxy-2-(6-phenethylamino-hexylamino)-ethyl]-1H-quinolin-2-on
- 5-[2-(2-{4-[4-(2-Amino-2-methyl-propoxy)-phenylamino]-phenyl}-ethylamino)-1-hydroxy-ethyl]-8-hydroxy-1H-quinolin-2-on
- [3-(4-{6-[2-Hydroxy-2-(4-hydroxy-3-hydroxymethyl-phenyl)-ethylamino]-hexyloxy}-butyl)-5-methyl-phenyl]-harnstoff
- 4-(2-{6-[2-(2,6-Dichloro-benzyloxy)-ethoxy]-hexylamino}-1-hydroxy-ethyl)-2-hydroxymethyl-phenol
- 3-(4-{6-[2-Hydroxy-2-(4-hydroxy-3-hydroxymethyl-phenyl)-ethylamino]-hexyloxy}-butyl)-benzylsulfonamid
- 3-(3-{7-[2-Hydroxy-2-(4-hydroxy-3-hydroxymethyl-phenyl)-ethylamino]-heptyloxy}-propyl)-benzylsulfonamid
- 4-(2-{6-[4-(3-Cyclopentanesulfonyl-phenyl)-butoxy]-hexylamino}-1-hydroxy-ethyl)-2-hydroxymethyl-phenol
- N-Adamantan-2-yl-2-(3-{2-[2-hydroxy-2-(4-hydroxy-3-hydroxymethyl-phenyl)-ethylamino]-propyl}-phenyl)-acetamid
gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind die Säureadditionssalze der Betamimetika ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als Anticholinergika gelangen hierbei vorzugsweise Verbindungen zur Anwendung, die ausgewählt sind aus der Gruppe bestehend aus Tiotropiumsalzen, bevorzugt das Bromidsalz, Oxitropiumsalzen, bevorzugt das Bromidsalz, Flutropiumsalzen, bevorzugt das Bromidsalz, Ipratropiumsalzen, bevorzugt das Bromidsalz, Glycopyrroniumsalzen, bevorzugt das Bromidsalz, Trospiumsalzen, bevorzugt das Chloridsalz, Tolterodin. In den vorstehend genannten Salzen stellen die Kationen die pharmakologisch aktiven Bestandteile dar. Als Anionen können die vorstehend genannten Salze bevorzugt enthalten Chlorid, Bromid, Iodid, Sulfat, Phosphat, Methansulfonat, Nitrat, Maleat, Acetat, Citrat, Fumarat, Tartrat, Oxalat, Succinat, Benzoat oder p-Toluolsulfonat, wobei Chlorid, Bromid, Iodid, Sulfat, Methansulfonat oder p-Toluolsulfonat als Gegenionen bevorzugt sind. Von allen Salzen sind die Chloride, Bromide, Iodide und Methansulfonate besonders bevorzugt.

Ebenfalls bevorzugte Anticholinergika sind ausgewählt aus den Salzen der Formel AC-1 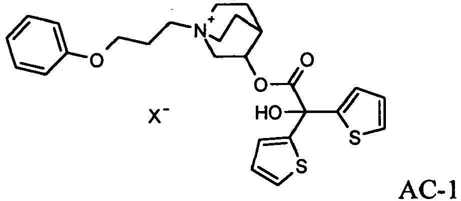 worin X⁻ ein einfach negativ geladenes Anion, bevorzugt ein Anion ausgewählt aus der Gruppe bestehend aus Fluorid, Chlorid, Bromid, Iodid, Sulfat, Phosphat, Methansulfonat, Nitrat, Maleat, Acetat, Citrat, Fumarat, Tartrat, Oxalat, Succinat, Benzoat und p-Toluolsulfonat, bevorzugt ein einfach negativ geladenes Anion, besonders bevorzugt ein Anion ausgewählt aus der Gruppe bestehend aus Fluorid, Chlorid, Bromid, Methansulfonat und p-Toluolsulfonat, insbesondere bevorzugt Bromid, bedeutet gegebenenfalls in Form ihrer Racemate, Enantiomere oder Hydrate. Von besonderer Bedeutung sind solche Arzneimittelkombinationen, die die Enantiomere der Formel AC-1-en 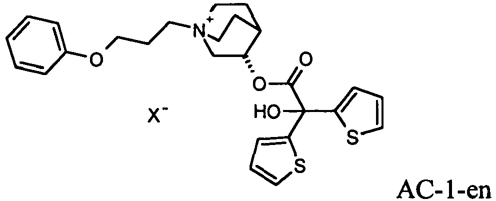 enthalten, worin X⁻ die vorstehend genannten Bedeutungen aufweisen kann. Weiterhin bevorzugte Anticholinergika sind ausgewählt aus den Salzen der Formel AC-2 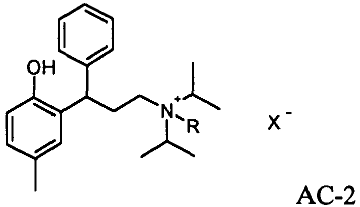 worin R entweder Methyl oder Ethyl bedeuten und worin X⁻ die vorstehend genannte Bedeutungen aufweisen kann. In einer alternativen Ausführungform kann die Verbindung der Formel AC-2 auch in Form der freien Base AC-2-base vorliegen.

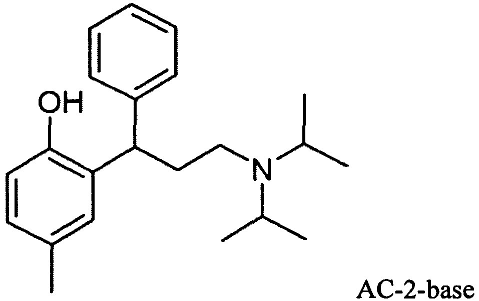

Weiterhin genannte Verbindungen sind:
- 2,2-Diphenylpropionsäuretropenolester-Methobromid
- 2,2-Diphenylpropionsäurescopinester-Methobromid
- 2-Fluor-2,2-Diphenylessigsäurescopinester-Methobromid
- 2-Fluor-2,2-Diphenylessigsäuretropenolester-Methobromid
- 3,3',4,4'-Tetrafluorbenzilsäuretropenolester-Methobromid
- 3,3',4,4'-Tetrafluorbenzilsäurescopinester-Methobromid
- 4,4'-Difluorbenzilsäuretropenolester-Methobromid
- 4,4'-Difluorbenzilsäurescopinester-Methobromid
- 3,3'-Difluorbenzilsäuretropenolester-Methobromid
- 3,3'-Difluorbenzilsäurescopinester-Methobromid
- 9-Hydroxy-fluoren-9-carbonsäuretropenolester-Methobromid
- 9-Fluor-fluoren-9-carbonsäuretropenolester-Methobromid
- 9-Hydroxy-fluoren-9-carbonsäurescopinester-Methobromid
- 9-Fluor-fluoren-9-carbonsäurescopinester-Methobromid
- 9-Methyl-fluoren-9-carbonsäuretropenolester-Methobromid
- 9-Methyl-fluoren-9-carbonsäurescopinester-Methobromid
- Benzilsäurecyclopropyltropinester-Methobromid
- 2,2-Diphenylpropionsäurecyclopropyltropinester-Methobromid
- 9-Hydroxy-xanthen-9-carbonsäurecyclopropyltropinester-Methobromid
- 9-Methyl-fluoren-9-carbonsäurecyclopropyltropinester-Methobromid
- 9-Methyl-xanthen-9-carbonsäurecyclopropyltropinester-Methobromid
- 9-Hydroxy-fluoren-9-carbonsäurecyclopropyltropinester-Methobromid
- 4,4'-Difluorbenzilsäuremethylestercyclopropyltropinester-Methobromid
- 9-Hydroxy-xanthen-9-carbonsäuretropenolester-Methobromid
- 9-Hydroxy-xanthen-9-carbonsäurescopinester-Methobromid
- 9-Methyl-xanthen-9-carbonsäuretropenolester-Methobromid
- 9-Methyl-xanthen-9-carbonsäurescopinester-Methobromid
- 9-Ethyl-xanthen-9-carbonsäuretropenolester-Methobromid
- 9-Difluormethyl-xanthen-9-carbonsäuretropenolester-Methobromid
- 9-Hydroxymethyl-xanthen-9-carbonsäurescopinester-Methobromid

Die vorstehend genannten Verbindungen sind im Rahmen der vorliegenden Erfindung auch als Salze einsetzbar, in denen statt des Methobromids, die Salze Metho-X zur Anwendung gelangen, wobei X die vorstehend für X⁻ genannten Bedeutungen haben kann.

Als Corticosteroide gelangen hierbei vorzugsweise Verbindungen zur Anwendung, die ausgewählt sind aus der Gruppe bestehend aus Beclomethason, Betamethason, Budesonid, Butixocort, Ciclesonid, Deflazacort, Dexamethason, Etiprednol, Flunisolid, Fluticason, Loteprednol, Mometason, Prednisolon, Prednison, Rofleponid, Triamcinolon, RPR-106541, NS-126, ST-26 und
- 6,9-Difluor-17-[(2-furanylcarbonyl)oxy]-11-hydroxy-16-methyl-3-oxo-androsta-1,4-dien-17-carbothionsäure (S)-fluoromethylester
- 6,9-Difluor-11-hydroxy-16-methyl-3-oxo-17-propionyloxy-androsta-1,4-dien-17-carbothionsäure (S)-(2-oxo-tetrahydro-furan-3S-yl)ester,
- 6α,9α-difluoro-11β-hydroxy-16α-methyl-3-oxo-17α-(2,2,3,3-tertamethylcyclopropylcarbonyl)oxy-androsta-1,4-diene-17β-carbonsäure cyanomethyl ester
gegebenenfalls in Form ihrer Racemate, Enantiomere oder Diastereomere und gegebenenfalls in Form ihrer Salze und Derivate, ihrer Solvate und/oder Hydrate. Jede Bezugnahme auf Steroide schließt eine Bezugnahme auf deren gegebenenfalls existierende Salze oder Derivate, Hydrate oder Solvate mit ein. Beispiele möglicher Salze und Derivate der Steroide können sein: Alkalisalze, wie beispielsweise Natrium- oder Kaliumsalze, Sulfobenzoate, Phosphate, I-sonicotinate, Acetate, Dichloroacetate, Propionate, Dihydrogenphosphate, Palmitate, Pivalate oder auch Furoate.

Als PDE4-Inhibitoren gelangen hierbei vorzugsweise Verbindungen zur Anwendung, die ausgewählt sind aus der Gruppe bestehend aus Enprofyllin, Theophyllin, Roflumilast, Ariflo (Cilomilast), Tofimilast, Pumafentrin, Lirimilast, Arofyllin, Atizoram, D-4418, Bay-198004, BY343, CP-325,366, D-4396 (Sch-351591), AWD-12-281 (GW-842470), NCS-613, CDP-840, D-4418, PD-168787, T-440, T-2585, V-11294A, Cl-1018, CDC-801, CDC-3052, D-22888, YM-58997, Z-15370 und
- N-(3,5-Dichloro-1-oxo-pyridin-4-yl)-4-difluormethoxy-3-cyclopropylmethoxybenzamid
- (-)p-[(4aR*,10bS*)-9-Ethoxy-1,2,3,4,4a,10b-hexahydro-8-methoxy-2-methylbenzo[s] [1,6]naphthyridin-6-yl]-N,N-diisopropylbenzamid
- (R)-(+)-1-(4-Brombenzyl)-4-[(3-cyclopentyloxy)-4-methoxyphenyl]-2-pyrrolidon
- 3-(Cyclopentyloxy-4-methoxyphenyl)-1-(4-N'-[N-2-cyano-S-methyl-isothioureido]benzyl)-2-pyrrolidon
- cis[4-Cyano-4-(3-cyclopentyloxy-4-methoxyphenyl)cyclohexan-1-carbonsäure]
- 2-carbomethoxy-4-cyano-4-(3-cyclopropylmethoxy-4-difluoromethoxy-phenyl)cyclohexan-1-on
- cis[4-Cyano-4-(3-cyclopropylmethoxy-4-difluormethoxyphenyl)cyclohexan-1-ol]
- (R)-(+)-Ethyl[4-(3-cyclopentyloxy-4-methoxyphenyl)pyrrolidin-2-yliden]acetat
- (S)-(-)-Ethyl[4-(3-cyclopentyloxy-4-methoxyphenyl)pyrrolidin-2-yliden]acetat
- 9-Cyclopentyl-5,6-dihydro-7-ethyl-3-(2-thienyl)-9H-pyrazolo[3,4-c]-1,2,4-triazolo[4,3-a]pyridin
- 9-Cyclopentyl-5,6-dihydro-7-ethyl-3-(tert-butyl)-9H-pyrazolo[3,4-c]-1,2,4-triazolo[4,3-a]pyridin
gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind die Säureadditionssalze der Betamimetika ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als LTD4-Antagonisten gelangen hierbei vorzugsweise Verbindungen zur Anwendung, die ausgewählt sind aus der Gruppe bestehend aus Montelukast, Pranlukast, Zafirlukast, MCC-847 (ZD-3523), MN-001, MEN-91507 (LM-1507), VUF-5078, VUF-K-8707, L-733321 und
- 1-(((R)-(3-(2-(6,7-Difluor-2-quinolinyl)ethenyl)phenyl)-3-(2-(2-hydroxy-2-propyl)phenyl)thio)methylcyclopropan-essigsäure,
- 1-(((1(R)-3(3-(2-(2,3-Dichlorthieno[3,2-b]pyridin-5-yl)-(E)-ethenyl)phenyl)-3-(2-(1-hydroxy-1-methylethyl)phenyl)-propyl)thio)methyl)cyclopropanessigsäure
- [2-[[2-(4-tert-Butyl-2-thiazolyl)-5-benzofuranyl]oxymethyl]phenyl]-essigsäure
gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind die Säureadditionssalze der Betamimetika ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat. Unter Salzen oder Derivaten zu deren Bildung die LTD4-Antagonisten gegebenenfalls in der Lage sind, werden beispielsweise verstanden: Alkalisalze, wie beispielsweise Natrium- oder Kaliumsalze, Erdalkalisalze, Sulfobenzoate, Phosphate, Isonicotinate, Acetate, Propionate, Dihydrogenphosphate, Palmitate, Pivalate oder auch Furoate.

Als EGFR-Hemmer gelangen hierbei vorzugsweise Verbindungen zur Anwendung, die ausgewählt sind aus der Gruppe bestehend aus Cetuximab, Trastuzumab, ABX-EGF, Mäb ICR-62 und
- 4-[(3-Chlor-4-fluorphenyl)amino]-6-{[4-(morpholin-4-yl)-1-oxo-2-buten-1-yl]amino}-7-cyclopropylmethoxy-chinazolin
- 4-[(3-Chlor-4-fluorphenyl)amino]-6-{[4-(N,N-diethylamino)-1-oxo-2-buten-1-yl]amino}-7-cyclopropylmethoxy-chinazolin
- 4-[(3-Chlor-4-fluorphenyl)amino]-6-{[4-(N,N-dimethylamino)-1-oxo-2-buten-1-yl]amino}-7-cyclopropylmethoxy-chinazolin
- 4-[(R)-(1-Phenyl-ethyl)amino]-6-{[4-(morpholin-4-yl)-1-oxo-2-buten-1-yl]amino}-7-cyclopentyloxy-chinazolin
- 4-[(3-Chlor-4-fluor-phenyl)amino]-6-{[4-((R)-6-methyl-2-oxo-morpholin-4-yl)-1-oxo-2-buten-1-yl]amino}-7-cyclopropylmethoxy-chinazolin
- 4-[(3-Chlor-4-fluor-phenyl)amino]-6-{[4-((R)-6-methyl-2-oxo-morpholin-4-yl)-1-oxo-2-buten-1-yl]amino}-7-[(S)-(tetrahydrofuran-3-yl)oxy]-chinazolin
- 4-[(3-Chlor-4-fluor-phenyl)amino]-6-{[4-((R)-2-methoxymethyl-6-oxo-morpholin-4-yl)-1-oxo-2-buten-1-yl]amino}-7-cyclopropylmethoxy-chinazolin
- 4-[(3-Chlor-4-fluor-phenyl)amino]-6-[2-((S)-6-methyl-2-oxo-morpholin-4-yl)-ethoxy]-7-methoxy-chinazolin
- 4-[(3-Chlor-4-fluorphenyl)amino]-6-({4-[N-(2-methoxy-ethyl)-N-methyl-amino]-1-oxo-2-buten-1-yl}amino)-7-cyclopropylmethoxy-chinazolin
- 4-[(3-Chlor-4-fluorphenyl)amino]-6-{[4-(N,N-dimethylamino)-1-oxo-2-buten-1-yl]amino}-7-cyclopentyloxy-chinazolin
- 4-[(R)-(1-Phenyl-ethyl)amino]-6-{[4-(N,N-bis-(2-methoxy-ethyl)-amino)-1-oxo-2-buten-1-yl]amino}-7-cyclopropylmethoxy-chinazolin
- 4-[(R)-(1-Phenyl-ethyl)amino]-6-({4-[N-(2-methoxy-ethyl)-N-ethyl-amino]-1-oxo-2-buten-1-yl}amino)-7-cyclopropylmethoxy-chinazolin
- 4-[(R)-(1-Phenyl-ethyl)amino]-6-({4-[N-(2-methoxy-ethyl)-N-methyl-amino]1-oxo-2-buten-1-yl}amino)-7-cyclopropylmethoxy-chinazolin
- 4-[(R)-(1-Phenyl-ethyl)amino]-6-({4-[N-(tetrahydropyran-4-yl)-N-methyl-amino]-1-oxo-2-buten-1-yl}amino)-7-cyclopropylmethoxy-chinazolin
- 4-[(3-Chlor-4-fluorphenyl)amino]-6-{[4-(N,N-dimethylamino)-1-oxo-2-buten-1-yl]amino}-7-((R)-tetrahydrofuran-3-yloxy)-chinazolin
- 4-[(3-Chlor-4-fluorphenyl)amino]-6-{[4-(N,N-dimethylamino)-1-oxo-2-buten-1-yl]amino}-7-((S)-tetrahydrofuran-3-yloxy)-chinazolin
- 4-[(3-Chlor-4-fluorphenyl)amino]-6-({4-[N-(2-methoxy-ethyl)-N-methyl-amino]-1-oxo-2-buten-1-yl}amino)-7-cyclopentyloxy-chinazolin
- 4-[(3-Chlor-4-fluorphenyl)amino]-6-{[4-(N-cyclopropyl-N-methyl-amino)-1-oxo-2-buten-1-yl]amino}-7-cyclopentyloxy-chinazolin
- 4-[(3-Chlor-4-fluorphenyl)amino]-6-{[4-(N,N-dimethylamino)-1-oxo-2-buten-1-yl]amino}-7-[(R)-(tetrahydrofuran-2-yl)methoxy]-chinazolin
- 4-[(3-Chlor-4-fluorphenyl)amino]-6-{[4-(N,N-dimethylamino)-1-oxo-2-buten-1-yl]amino}-7-[(S)-(tetrahydrofuran-2-yl)methoxy]-chinazolin
- 4-[(3-Ethinyl-phenyl)amino]-6,7-bis-(2-methoxy-ethoxy)-chinazolin
- 4-[(3-Chlor-4-fluorphenyl)amino]-7-[3-(morpholin-4-yl)-propyloxy]-6-[(vinylcarbonyl)amino]-chinazolin
- 4-[(R)-(1-Phenyl-ethyl)amino]-6-(4-hydroxy-phenyl)-7H-pyrrolo[2,3-d]pyrimidin
- 3-Cyano-4-[(3-chlor-4-fluorphenyl)amino]-6-{[4-(N,N-dimethylamino)-1-oxo-2-buten-1-yl]amino}-7-ethoxy-chinolin
- 4-{[3-Chlor-4-(3-fluor-benzyloxy)-phenyl]amino}-6-(5-{[(2-methansulfonyl-ethyl)amino]methyl}-furan-2-yl)chinazolin
- 4-[(R)-(1-Phenyl-ethyl)amino]-6-{[4-((R)-6-methyl-2-oxo-morpholin-4-yl)-1-oxo-2-buten-1-yl]amino}-7-methoxy-chinazolin
- 4-[(3-Chlor-4-fluorphenyl)amino]-6-{[4-(morpholin-4-yl)-1-oxo-2-buten-1-yl] amino}-7-[(tetrahydrofuran-2-yl)methoxy]-chinazolin
- 4-[(3-Chlor-4-fluorphenyl)amino]-6-({4-[N,N-bis-(2-methoxy-ethyl)-amino]-1-oxo-2-buten-1-yl}amino)-7-[(tetrahydrofuran-2-yl)methoxy]-chinazolin
- 4-[(3-Ethinyl-phenyl)amino]-6-{[4-(5,5-dimethyl-2-oxo-morpholin-4-yl)-1-oxo-2-buten-1-yl]amino}-chinazolin
- 4-[(3-Chlor-4-fluor-phenyl)amino]-6-[2-(2,2-dimethyl-6-oxo-morpholin-4-yl)-ethoxy]-7-methoxy-chinazolin
- 4-[(3-Chlor-4-fluor-phenyl)amino]-6-[2-(2,2-dimethyl-6-oxo-morpholin-4-yl)-ethoxy]-7-[(R)-(tetrahydrofuran-2-yl)methoxy]-chinazolin
- 4-[(3-Chlor-4-fluor-phenyl)amino]-7-[2-(2,2-dimethyl-6-oxo-morpholin-4-yl)-ethoxy]-6-[(S)-(tetrahydrofuran-2-yl)methoxy]-chinazolin
- 4-[(3-Chlor-4-fluor-phenyl)amino]-6-{2-[4-(2-oxo-morpholin-4-yl)-piperidin-1-yl]-ethoxy}-7-methoxy-chinazolin
- 4-[(3-Chlor-4-fluor-phenyl)amino]-6-[1-(tert.-butyloxycarbonyl)-piperidin-4-yloxy]-7-methoxy-chinazolin
- 4-[(3-Chlor-4-fluor-phenyl)amino]-6-(trans-4-amino-cyclohexan-1-yloxy)-7-methoxy-chinazolin
- 4-[(3-Chlor-4-fluor-phenyl)amino]-6-(trans-4-methansulfonylamino-cyclohexan-1-yloxy)-7-methoxy-chinazolin
- 4-[(3-Chlor-4-fluor-phenyl)amino]-6-(tetrahydropyran-3-yloxy)-7-methoxy-chinazolin
- 4-[(3-Chlor-4-fluor-phenyl)amino]-6-(1-methyl-piperidin-4-yloxy)-7-methoxy-chinazolin
- 4-[(3-Chlor-4-fluor-phenyl)amino]-6-{1-[(morpholin-4-yl)carbonyl]-piperidin-4-yloxy}-7-methoxy-chinazolin
- 4-[(3-Chlor-4-fluor-phenyl)amino]-6-{1-[(methoxymethyl)carbonyl]-piperidin-4-yloxy}-7-methoxy-chinazolin
- 4-[(3-Chlor-4-fluor-phenyl)amino]-6-(piperidin-3-yloxy)-7-methoxy-chinazolin
- 4-[(3-Chlor-4-fluor-phenyl)amino]-6-[1-(2-acetylamino-ethyl)-piperidin-4-yloxy]-7-methoxy-chinazolin
- 4-[(3-Chlor-4-fluor-phenyl)amino]-6-(tetrahydropyran-4-yloxy)-7-ethoxy-chinazolin
- 4-[(3-Chlor-4-fluor-phenyl)amino]-6-((S)-tetrahydrofuran-3-yloxy)-7-hydroxy-chinazolin
- 4-[(3-Chlor-4-fluor-phenyl)amino]-6-(tetrahydropyran-4-yloxy)-7-(2-methoxy-ethoxy)-chinazolin
- 4-[(3-Chlor-4-fluor-phenyl)amino]-6-{trans-4-[(dimethylamino)sulfonylamino]-cyclohexan-1-yloxy}-7-methoxy-chinazolin
- 4-[(3-Chlor-4-fluor-phenyl)amino]-6-{trans-4-[(morpholin-4-yl)carbonylamino]-cyclohexan-1-yloxy}-7-methoxy-chinazolin
- 4-[(3-Chlor-4-fluor-phenyl)amino]-6-{trans-4-[(morpholin-4-yl)sulfonylamino]-cyclohexan-1-yloxy}-7-methoxy-chinazolin
- 4-[(3-Chlor-4-fluor-phenyl)amino]-6-(tetrahydropyran-4-yloxy)-7-(2-acetylamino-ethoxy)-chinazolin
- 4-[(3-Chlor-4-fluor-phenyl)amino]-6-(tetrahydropyran-4-yloxy)-7-(2-methansulfonylamino-ethoxy)-chinazolin
- 4-[(3-Chlor-4-fluor-phenyl)amino]-6-{1-[(piperidin-1-yl)carbonyl]-piperidin-4-yloxy}-7-methoxy-chinazolin
- 4-[(3-Chlor-4-fluor-phenyl)amino]-6-(1-aminocarbonylmethyl-piperidin-4-yloxy)-7-methoxy-chinazolin
- 4-[(3-Chlor-4-fluor-phenyl)amino]-6-(cis-4-{N-[(tetrahydropyran-4-yl)carbonyl]-N-methyl-amino}-cyclohexan-1-yloxy)-7-methoxy-chinazolin
- 4-[(3-Chlor-4-fluor-phenyl)amino]-6-(cis-4-{N-[(morpholin-4-yl)carbonyl]-N-methyl-amino}-cyclohexan-1-yloxy)-7-methoxy-chinazolin
- 4-[(3-Chlor-4-fluor-phenyl)amino]-6-(cis-4-{N-[(morpholin-4-yl)sulfonyl]-N-methyl-amino}-cyclohexan-1-yloxy)-7-methoxy- chinazolin
- 4-[(3-Chlor-4-fluor-phenyl)amino]-6-(trans-4-ethansulfonylamino-cyclohexan-1-yloxy)-7-methoxy-chinazolin
- 4-[(3-Chlor-4-fluor-phenyl)amino]-6-(1-methansulfonyl-piperidin-4-yloxy)-7-ethoxy-chinazolin
- 4-[(3-Chlor-4-fluor-phenyl)amino]-6-(1-methansulfonyl-piperidin-4-yloxy)-7-(2-methoxy-ethoxy)-chinazolin
- 4-[(3-Chlor-4-fluor-phenyl)amino]-6-[1-(2-methoxy-acetyl)-piperidin-4-yloxy]-7-(2-methoxy-ethoxy)-chinazolin
- 4-[(3-Chlor-4-fluor-phenyl)amino]-6-(cis-4-acetylamino-cyclohexan-1-yloxy)-7-methoxy-chinazolin
- 4-[(3-Ethinyl-phenyl)amino]-6-[1-(tert.-butyloxycarbonyl)-piperidin-4-yloxy]-7-methoxy-chinazolin
- 4-[(3-Ethinyl-phenyl)amino]-6-(tetrahydropyran-4-yloxy]-7-methoxy-chinazolin
- 4-[(3-Chlor-4-fluor-phenyl)amino]-6-(cis-4-{N-[(piperidin-1-yl)carbonyl]-N-methyl-amino}-cyclohexan-1-yloxy)-7-methoxy-chinazolin
- 4-[(3-Chlor-4-fluor-phenyl)amino]-6-(cis-4-{N-[(4-methyl-piperazin-1-yl)carbonyl]-N-methyl-amino}-cyclohexan-1-yloxy)-7-methoxy-chinazolin
- 4-[(3-Chlor-4-fluor-phenyl)amino]-6-{cis-4-[(morpholin-4-yl)carbonylamino]-cyclohexan-1-yloxy}-7-methoxy-chinazolin
- 4-[(3-Chlor-4-fluor-phenyl)amino]-6-{1-[2-(2-oxopyrrolidin-1-yl)ethyl]-piperidin-4-yloxy}-7-methoxy-chinazolin
- 4-[(3-Chlor-4-fluor-phenyl)amino]-6-{1-[(morpholin-4-yl)carbonyl]-piperidin-4-yloxy}-7-(2-methoxy-ethoxy)-chinazolin
- 4-[(3-Ethinyl-phenyl)amino]-6-(1-acetyl-piperidin-4-yloxy)-7-methoxy-chinazolin
- 4-[(3-Ethinyl-phenyl)amino]-6-(1-methyl-piperidin-4-yloxy)-7-methoxy-chinazolin
- 4-[(3-Ethinyl-phenyl)amino]-6-(1-methansulfonyl-piperidin-4-yloxy)-7-methoxy-chinazolin
- 4-[(3-Chlor-4-fluor-phenyl)amino]-6-(1-methyl-piperidin-4-yloxy)-7(2-methoxy-ethoxy)-chinazolin
- 4-[(3-Chlor-4-fluor-phenyl)amino]-6-(1-isopropyloxycarbonyl-piperidin-4-yloxy)-7-methoxy-chinazolin
- 4-[(3-Chlor-4-fluor-phenyl)amino]-6-(cis-4-methylamino-cyclohexan-1-yloxy)-7-methoxy-chinazolin
- 4-[(3-Chlor-4-fluor-phenyl)amino]-6-{cis-4-[N-(2-methoxy-acetyl)-N-methyl-amino]-cyclohexan-1-yloxy}-7-methoxy-chinazolin
- 4-[(3-Ethinyl-phenyl)amino]-6-(piperidin-4-yloxy)-7-methoxy-chinazolin
- 4-[(3-Ethinyl-phenyl)amino]-6-[1-(2-methoxy-acetyl)-piperidin-4-yloxy]-7-methoxy-chinazolin
- 4-[(3-Ethinyl-phenyl)amino]-6-{1-[(morpholin-4-yl)carbonyl]-piperidin-4-yloxy}-7-methoxy-chinazolin
- 4-[(3-Chlor-4-fluor-phenyl)amino]-6-{1-[(cis-2,6-dimethyl-morpholin-4-yl)carbonyl]-piperidin-4-yloxy}-7-methoxy-chinazolin
- 4-[(3-Chlor-4-fluor-phenyl)amino]-6-{1-[(2-methyl-morpholin-4-yl)carbonyl]-piperidin-4-yloxy}-7-methoxy-chinazolin
- 4-[(3-Chlor-4-fluor-phenyl)amino]-6-{1-[(S,S)-(2-oxa-5-aza-bicyclo[2.2.1]hept-5-yl)carbonyl]-piperidin-4-yloxy}-7-methoxy-chinazolin
- 4-[(3-Chlor-4-fluor-phenyl)amino]-6-{1-[(N-methyl-N-2-methoxyethyl-amino)carbonyl]-piperidin-4-yloxy}-7-methoxy-chinazolin
- 4-[(3-Chlor-4-fluor-phenyl)amino]-6-(1-ethyl-piperidin-4-yloxy)-7-methoxy-chinazolin
- 4-[(3-Chlor-4-fluor-phenyl)amino]-6-{1-[(2-methoxyethyl)carbonyl]-piperidin-4-yloxy}-7-methoxy-chinazolin
- 4-[(3-Chlor-4-fluor-phenyl)amino]-6-{1-[(3-methoxypropyl-amino)-carbonyl]-piperidin-4-yloxy}-7-methoxy-chinazolin
- 4-[(3-Chlor-4-fluor-phenyl)amino]-6-[cis-4-(N-methansulfonyl-N-methyl-amino)-cyclohexan-1-yloxy]-7-methoxy-chinazolin
- 4-[(3-Chlor-4-fluor-phenyl)amino]-6-[cis-4-(N-acetyl-N-methyl-amino)-cyclohexan-1-yloxy]-7-methoxy-chinazolin
- 4-[(3-Chlor-4-fluor-phenyl)amino]-6-(trans-4-methylamino-cyclohexan-1-yloxy)-7-methoxy-chinazolin
- 4-[(3-Chlor-4-fluor-phenyl)amino]-6-[trans-4-(N-methansulfonyl-N-methyl-amino)-cyclohexan-1-yloxy]-7-methoxy-chinazolin
- 4-[(3-Chlor-4-fluor-phenyl)amino]-6-(trans-4-dimethylamino-cyclohexan-1-yloxy)-7-methoxy-chinazolin
- 4-[(3-Chlor-4-fluor-phenyl)amino]-6-(trans-4-{N-[(morpholin-4-yl)carbonyl]-N-methyl-amino}-cyclohexan-1-yloxy)-7-methoxy-chinazolin
- 4-[(3-Chlor-4-fluor-phenyl)amino]-6-[2-(2,2-dimethyl-6-oxo-morpholin-4-yl)-ethoxy]-7-[(S)-(tetrahydrofuran-2-yl)methoxy]-chinazolin
- 4-[(3-Chlor-4-fluor-phenyl)amino]-6-(1-methansulfonyl-piperidin-4-yloxy)-7-methoxy-chinazolin
- 4-[(3-Chlor-4-fluor-phenyl)amino]-6-(1-cyano-piperidin-4-yloxy)-7-methoxy-chinazolin
gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind die Säureadditionssalze der Betamimetika ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als Dopamin-Agonisten gelangen hierbei vorzugsweise Verbindungen zur Anwendung, die ausgewählt sind aus der Gruppe bestehend aus Bromocriptin, Cabergolin, Alpha-Dihydroergocryptin, Lisurid, Pergolid, Pramipexol, Roxindol, Ropinirol, Talipexol, Tergurid und Viozan, gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind die Säureadditionssalze der Betamimetika ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als H1-Antihistaminika gelangen hierbei vorzugsweise Verbindungen zur Anwendung, die ausgewählt sind aus der Gruppe bestehend aus Epinastin, Cetirizin, Azelastin, Fexofenadin, Levocabastin, Loratadin, Mizolastin, Ketotifen, Emedastin, Dimetinden; Clemastin, Bamipin, Cexchlorpheniramin, Pheniramin, Doxylamin, Chlorphenoxamin, Dimenhydrinat, Diphenhydramin, Promethazin, Ebastin, Desloratidin und Meclozin, gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind die Säureadditionssalze der Betamimetika ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Außerdem können inhalierbare Makromoleküle verwendet werden, wie in EP 1 003 478 A1 oder CA 2297174 A1 offenbart.

Weiterhin kann die Verbindung aus der Gruppe der Derivate von Mutterkornalkaloiden, der Triptane, der CGRP-Hemmern, der Phosphodiesterase-V-Hemmer stammen, gegebenenfalls in Form ihrer Racemate, Enantiomere oder Diastereomere, gegebenenfalls in Form ihrer pharmakologisch verträglichen Säureadditionssalze, ihrer Solvate und/oder Hydrate.

Als Derivate der Mutterkornalkaloide: Dihydroergotamin, Ergotamin.

**Bezugszeichenliste**

| | | | |
|---|---|---|---|
| 1 | Inhalationsvorrichtung | 38 | Anstechelement |
| 2 | Aerosol | | |
| 3 | Inhalator | | |
| 4 | Anschlußeinrichtung | | |
| 5 | Anschlußabschnitt | | |
| 6 | Mundstück | | |
| 7 | Aufnahmeraum | | |
| 8 | Behältnis | | |
| 9 | Verbindungsabschnitt | | |
| 10 | Befestigungsmittel | | |
| 11 | freies Ende | | |
| 12 | Verbindungsnaht | | |
| 13 | Flachseiten | | |
| 14 | Ventil- bzw. Steuereinrichtung | | |
| 15 | Gehäuse | | |
| 16 | Kante | | |
| 17 | Zuschnitt | | |
| 18 | Linie | | |
| 19 | Längsrand | | |
| 20 | Arzneimittelzubereitung | | |
| 21 | Behälter | | |
| 22 | Beutel | | |
| 23 | Druckerzeuger | | |
| 24 | Halterung | | |
| 25 | Antriebsfeder | | |
| 26 | Sperrelement | | |
| 27 | Förderrohr | | |
| 28 | Rückschlagventil | | |
| 29 | Druckkammer | | |
| 30 | Austragsdüse | | |
| 31 | Zuluftöffnung | | |
| 32 | erstes Gehäuseteil (Oberteil) | | |
| 33 | Innenteil | | |
| 33a | oberer Teil des Innenteils | | |
| 33b | unterer Teil des Innenteils | | |
| 34 | zweites Gehäuseteil (Unterteil) | | |
| 35 | Halteelement | | |
| 36 | Feder (im Gehäuseunterteil) | | |
| 37 | Behälterboden | | |

## Patentansprüche

1. Inhalationsvorrichtung (1) zur Zwischenspeicherung eines Aerosols (2), mit einer Anschlußeinrichtung und einem daran angeschlossenen oder anschließbaren, beim Inhalieren kollabierenden Behältnis (8), das derartig ausgebildet ist, dass es beim Kollabieren seine Länge zumindest im wesentlichen beibehält, wobei das Behältnis (8) zum Kollabieren längs gefaltet oder längs faltbar ist,
**dadurch gekennzeichnet,**
**daß** das Behältnis (8) zumindest im wesentlichen keilförmig ausgebildet ist und sich der Querschnitts des Behältnisses (8) zu einem freien Ende (11) hin kontinuierlich verjüngt.

2. Inhalationsvorrichtung nach Anspruch 1, **dadurch gekennzeichnet, daß** das Behältnis (8) zumindest bereichsweise flach ausgebildet ist.

3. Inhalationsvorrichtung nach Anspruch 1 oder 2, **dadurch gekennzeichnet, daß** das Behältnis (8) ein flaches freies Ende (11) aufweist.

4. Inhalationsvorrichtung nach einem der voranstehenden Ansprüche, **dadurch gekennzeichnet, daß** das Behältnis (8) aus einem Zuschnitt (17) hergestellt ist.

5. Inhalationsvorrichtung nach einem der voranstehenden Ansprüche, **dadurch gekennzeichnet, daß** das Behältnis (8) aus einem flexiblen Material besteht oder hergestellt ist.

6. Inhalationsvorrichtung nach einem der voranstehenden Ansprüche, **dadurch gekennzeichnet, daß** das Behältnis (8) aus einem weichen Material besteht oder hergestellt ist.

7. Inhalationsvorrichtung nach Anspruch 5 oder 6, **dadurch gekennzeichnet, daß** das Behältnis (8) aus Folie oder aus Papier besteht oder hergestellt ist.

8. Inhalationsvorrichtung nach einem der voranstehenden Ansprüche, **dadurch gekennzeichnet, daß** das Behältnis (8) wechselbar ist.

9. Inhalationsvorrichtung nach einem der voranstehenden Ansprüche, **dadurch gekennzeichnet, daß** das Behältnis (8) mit der Anschlußeinrichtung (4) klemmend verbunden oder verbindbar ist.

10. Inhalationsvorrichtung nach einem der voranstehenden Ansprüche, **dadurch gekennzeichnet, daß** das Behältnis (8) lösbar mit der Anschlußeinrichtung (4) verbunden oder verbindbar ist.

11. Inhalationsvorrichtung nach einem der voranstehenden Ansprüche, **dadurch gekennzeichnet, daß** das Behältnis (8) mittels eines elastischen Befestigungsmittels (10), wie eines Gummibands oder eines O-Rings, mit der Anschlußeinrichtung (4) verbindbar ist.

12. Inhalationsvorrichtung nach einem der voranstehenden Ansprüche, **dadurch gekennzeichnet, daß** das Behältnis (8) mittels eines ringförmigen Befestigungsmittels (10) mit der Anschlußeinrichtung (4) verbindbar ist.

13. Inhalationsvorrichtung nach einem der voranstehenden Ansprüche, **dadurch gekennzeichnet, daß** die Anschlußeinrichtung einen stutzenartigen Verbindungsabschnitt (9) zur Halterung des Behältnisses (8) aufweist.

14. Inhalationsvorrichtung nach einem der voranstehenden Ansprüche, **dadurch gekennzeichnet, daß** die Anschlußeinrichtung (4) einen Anschlußabschnitt (5) aufweist oder bildet.

15. Inhalationsvorrichtung nach einem der voranstehenden Ansprüche, **dadurch gekennzeichnet, daß** die Inhalationsvorrichtung (1) ein Gehäuse (15) für das Behältnis (8) aufweist.

## Claims

1. Inhalation device (1) for the intermediate storage of an aerosol (2), having a connecting device and a container (8) that is connected or connectable thereto and that collapses during inhalation and is configured so that as it collapses it maintains its length at least substantially, the container (8) being folded longitudinally or foldable longitudinally for collapsing,
**characterised in that**
the container (8) is at least substantially wedge-shaped in construction and
the cross-section of the container (8) tapers continuously towards a free end (11).

2. Inhalation device according to claim 1, **characterised in that** the container (8) is configured to be flat, at least in parts.

3. Inhalation device according to claim 1 or 2, **characterised in that** the container (8) has a flat free end (11).

4. Inhalation device according to one of the preceding claims, **characterised in that** the container (8) is made from a blank (17).

5. Inhalation device according to one of the preceding claims, **characterised in that** the container (8) consists of or is made from a flexible material.

6. Inhalation device according to one of the preceding claims, **characterised in that** the container (8) consists of or is made from a soft material.

7. Inhalation device according to claim 5 or 6, **characterised in that** the container (8) consists of or is made from film or paper.

8. Inhalation device according to one of the preceding claims, **characterised in that** the container (8) is replaceable.

9. Inhalation device according to one of the preceding claims, **characterised in that** the container (8) is connected or connectable to the connecting device (4) by a clamping action.

10. Inhalation device according to one of the preceding claims, **characterised in that** the container (8) is releasably connected or connectable to the connecting device (4).

11. Inhalation device according to one of the preceding claims, **characterised in that** the container (8) can be connected to the connecting device (4) by means of an elastic attachment means (10) such as a rubber band or an O-ring.

12. Inhalation device according to one of the preceding claims, **characterised in that** the container (8) can be connected to the connecting device (4) by means of an annular attachment means (10).

13. Inhalation device according to one of the preceding claims, **characterised in that** the connecting device has a tube-like connecting portion (9) for mounting the container (8).

14. Inhalation device according to one of the preceding claims, **characterised in that** the connecting device (4) comprises or forms an attachment portion (5).

15. Inhalation device according to one of the preceding claims, **characterised in that** the inhalation device (1) has a housing (15) for the container (8).

## Revendications

1. Système d'inhalation (1) pour le stockage provisoire d'un aérosol (2), comportant un dispositif de connexion et un récipient (8) collaborant à l'inhalation qui lui est raccordé ou peut lui être raccordé, le récipient (8) étant réalisé de manière à conserver au moins sensiblement sa longueur au cours de la collaboration, le récipient (8) adapté pour collaborer étant plié ou pouvant être plié dans le sens de la longueur,
**caractérisé en ce que**
le récipient (8) est prévu sensiblement en forme de biseau et **en ce que** la section transversale du récipient (8) s'amincit de manière continue vers une extrémité libre (11).

2. Système d'inhalation selon la revendication 1, **caractérisé en ce que** le récipient (8) est prévu au moins partiellement plat.

3. Système d'inhalation selon la revendication 1 ou 2, **caractérisé en ce que** le récipient (8) présente une extrémité libre (11) plate.

4. Système d'inhalation selon l'une des revendications précédentes, **caractérisé en ce que** le récipient (8) est fabriqué à partir d'une découpe (17).

5. Système d'inhalation selon l'une des revendications précédentes, **caractérisé en ce que** le récipient (8) est constitué d'un matériau souple ou est fabriqué avec un matériau souple.

6. Système d'inhalation selon l'une des revendications précédentes, **caractérisé en ce que** le récipient (8) est constitué d'un matériau mou ou est fabriqué avec un matériau mou.

7. Système d'inhalation selon la revendication 5 ou 6, **caractérisé en ce que** le récipient (8) est constitué d'une feuille ou de papier ou est fabriqué avec une feuille ou en papier.

8. Système d'inhalation selon l'une des revendications précédentes, **caractérisé en ce que** le récipient (8) peut être changé.

9. Système d'inhalation selon l'une des revendications précédentes, **caractérisé en ce que** le récipient (8) est raccordé ou peut être raccordé par serrage au dispositif de connexion (4).

10. Système d'inhalation selon l'une des revendications précédentes, **caractérisé en ce que** le récipient (8) est raccordé ou peut être raccordé de manière amovible au dispositif de connexion (4).

11. Système d'inhalation selon l'une des revendications précédentes, **caractérisé en ce que** le récipient (8) peut être raccordé au dispositif de connexion (4) au moyen d'un élément de fixation élastique (10), tel qu'une bande en caoutchouc ou un joint torique.

12. Système d'inhalation selon l'une des revendications précédentes, **caractérisé en ce que** le récipient (8) peut être raccordé au dispositif de connexion (4) au moyen d'un élément de fixation annulaire (10).

13. Système d'inhalation selon l'une des revendications précédentes, **caractérisé en ce que** le dispositif de connexion comporte un segment de raccordement (9) en forme de tubulure pour le maintien du récipient (8).

14. Système d'inhalation selon l'une des revendications précédentes, **caractérisé en ce que** le dispositif de connexion (4) présente ou constitue une section de connexion (5).

15. Système d'inhalation selon l'une des revendications précédentes, **caractérisé en ce que** le système d'inhalation (1) comporte un boîtier (15) pour le récipient (8).
